# EUROPEAN PATENT SPECIFICATION

(11) **EP 4 412 534 B1**
(45) Date of publication and mention of the grant of the patent: **02.09.2026**
(21) Application number: 21794741.5
(22) Date of filing: 08.10.2021
(51) Int. Cl.: A61B 17/00, A61B 17/12, A61M 5/145

(54) **SYSTEMS FOR USING HYDRAULIC FORCES FOR DELIVERING FLOWABLE, THERAPEUTIC COMPOSITIONS TO SURGICAL SITES**
SYSTEME ZUR VERWENDUNG HYDRAULISCHER KRÄFTE ZUR ABGABE VON FLIESSFÄHIGEN THERAPEUTISCHEN ZUSAMMENSETZUNGEN AN CHIRURGISCHE STELLEN
SYSTÈMES D'UTILISATION DE FORCES HYDRAULIQUES POUR ADMINISTRER DES COMPOSITIONS THÉRAPEUTIQUES POUVANT S'ÉCOULER À DES SITES CHIRURGICAUX

(43) Date of publication of application: 14.08.2024
(73) Proprietor: Guangzhou Bioseal Biotech Co., Ltd., Guangzhou, Guangdong 510530 (CN); Ethicon, Inc., Raritan, NJ 08869 (US)
(72) Inventor: XIE, Shiwei, Guangzhou, Guangdong 510530 (CN); GUO, Jianxin, Raritan, New Jersey 08869 (US); GUO, Xuelin, Raritan, New Jersey 08869 (US)
(74) Representative: Carpmaels & Ransford LLP
(86) International application number: PCT/CN2021/122569
(87) International publication number: WO 2023/056588

(56) References cited:
- EP-A2- 2 822 474
- WO-A1-01/21234
- WO-A1-01/39669
- WO-A1-2004/067065
- WO-A1-2007/010522
- WO-A1-2017/021850
- WO-A1-2017/079136
- DE-A1- 19 623 779
- DE-A1- 4 106 624
- US-A1- 2007 264 130
- US-A1- 2014 276 415
- US-A1- 2014 276 582
- ANONYMOUS: "Cannula - Wikipedia", 22 July 2021 (2021-07-22), en.wikipedia.org, pages 1 - 6, XP055901810, Retrieved from the Internet <URL:https://en.wikipedia.org/w/index.php?title=Cannula&oldid=1034866078> [retrieved on 20220316]

## Description

### BACKGROUND OF THE INVENTION

### Field of the Invention

The present patent is generally related to systems for controlling bleeding at surgical sites and is more particularly related to systems to be used for delivering flowable, therapeutic compositions, such as flowable hemostats, for controlling bleeding.

### Description of the Related Art

Protein-based hemostatic materials such as collagen and gelatin are commercially available in solid sponge and loose or unpacked powder form for use in surgical procedures. Mixing of the loose or unpacked powder with a fluid such as saline or a thrombin solution may form a paste or slurry that is beneficial as a hemostatic composition for use in cases of diffuse bleeding, particularly from uneven surfaces or hard to reach areas, depending on mixing conditions and relative ratios of the materials.

Conventional hemostatic pastes are usually prepared at the point of use by mechanical agitation and mixing of a biocompatible polymer (e.g., a gelatin), and a liquid (e.g., a thrombin solution), to provide uniformity to the composition. Mixing to form a paste usually requires extensive mixing, such as kneading or transfer between two syringes.

It is often desirable that the hemostatic paste includes a thrombin component to enhance the optimal hemostatic effect of the paste. Due to stability reasons, the thrombin component is usually provided as a dry composition separate from the biocompatible polymer component. The dry thrombin is then reconstituted to form a suspension or solution before mixing with the biocompatible polymer. The reconstitution step of the thrombin component usually takes place immediately prior to mixing with the biocompatible polymer. Reconstitution of thrombin is time consuming and challenging with multi-step syringe handlings involved; factors which are undesirable in an operating room setting with bleedings, as the surgeon will have to pause the procedure while waiting for the hemostat to be prepared.

In view of the above-noted deficiencies, there have been efforts directed to providing safe and effective flowable hemostats that are used for controlling bleeding. For example, the SURGIFLO^{®} Hemostatic Matrix Kit with Thrombin, sold by Ethicon, Inc. of Somerville, New Jersey, is a kit that is used for producing a hemostatic gelatin paste including thrombin, which is prepared by first reconstituting a dry thrombin composition and subsequently transferring a gelatin matrix-thrombin solution mixture back and forth between two connected syringes for a total of at least six passes.

Other attempts have been made to provide the biocompatible polymer and the thrombin in dry form in the same syringe, such as described in WO 2011/151400, WO 2011/151384, WO 2011/151386 and WO 2013/185776, the disclosures of which are hereby incorporated by reference herein. Due to the sensitivity of thrombin to the sterilization methods usually employed in the manufacture of hemostatic products (i.e., ionizing radiation and/or ethylene oxide), the sensitivity of thrombin to water and the different physical-chemical properties of thrombin and the polymers usually employed, such as gelatin, it has proven challenging to manufacture such "all-in-one" products so that they retain sufficient thrombin activity during manufacturing, sterilization and throughout the shelf-life of the product and/or which ensures satisfactory distribution of thrombin in the final reconstituted hemostatic paste product.

As noted above, incorporating thrombin into hemostatic pastes has proven to be challenging due to either manufacturing or stability reasons or due to time consuming reconstitution of a dry thrombin composition prior to mixing with the biocompatible polymer. Thus, there has been a long-felt need in the art for developing novel methods for quickly and easily incorporating thrombin into a hemostatic paste, and for simple and fast methods for preparing a hemostatic composition in the operating room where potential bleeding must be controlled in a fast and efficient manner.

In response, US 2019/0343981, assigned to Ethicon, Inc. of Somerville, New Jersey, the disclosure of which is hereby incorporated by reference herein, teaches a method for preparing a hemostatic composition that includes thrombin. In one embodiment, the method includes the step of reconstituting a dry thrombin directly in a paste, such as a paste comprising a biocompatible polymer. The hemostatic composition including the thrombin may be prepared from a dry thrombin composition and a paste in a single step. The flowable hemostat is used for treating a wound (e.g., controlling bleeding).

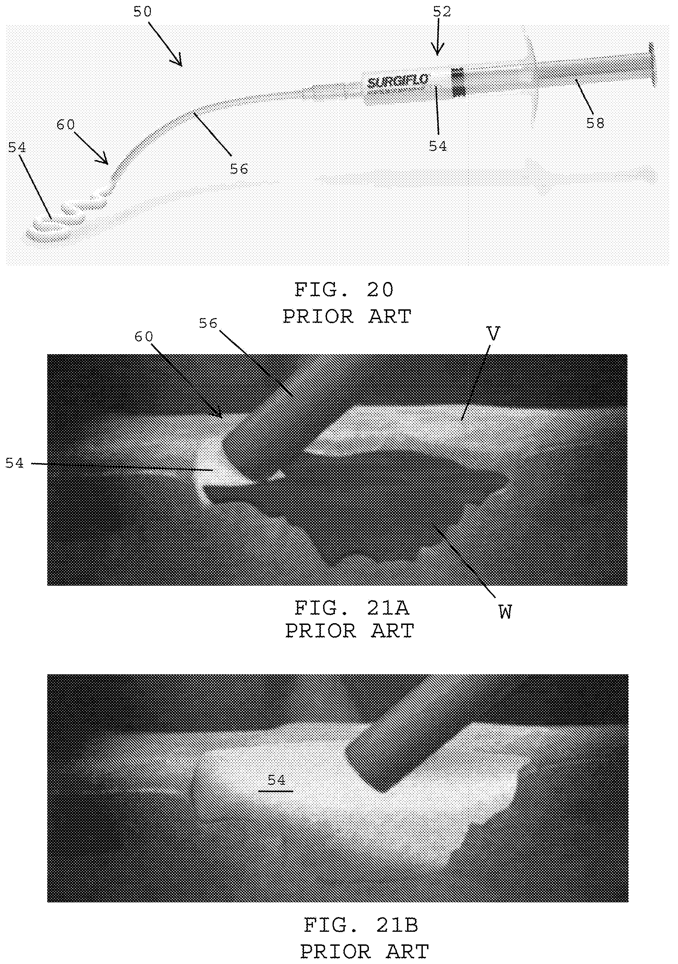

Hemostatic pastes may be expressed using a pumping system, such as a syringe. Referring to FIG. 20, one prior art system for controlling bleeding at a surgical site includes an applicator device 50 for dispensing a flowable hemostat, such as the flowable hemostat matrix with Thrombin sold under the trademark SURGIFLO^{®}, by Ethicon, Inc. of Somerville, New Jersey. The applicator device 50 includes a syringe 52 that contains a flowable hemostat 54 and an applicator tip 56 that is secured to a distal end of the syringe 52. A syringe plunger 58 may be depressed for dispensing the flowable hemostat 54 from a distal end 60 of the applicator tip 56.

The system shown in FIG. 20 may be used for controlling bleeding. In FIG. 21A, a vessel V has a wound W that is bleeding. The distal end 60 of the applicator tip 56 is juxtaposed with the bleeding wound site to apply the flowable hemostat 54 over the wound W.

FIG. 21B shows the bleeding wound site after it has been fully covered by a mass of the flowable hemostat 54. The flowable hemostat helps in blood clotting by speeding up the conversion of a substance in the blood called fibrin, to fibrinogen. This chemical reaction leads to the formation of blood clots, which is the final step in the process of hemostasis.

In some instances, a residual amount of the flowable hemostat remains within the cannula of the applicator tip 56. To eliminate waste and use a higher percentage of the flowable hemostat that has been prepared, it is desirable if the residual amount of the flowable hemostat that remains within the applicator tip 56 can be dispensed from the distal end 60 of the dispensing device. In some instances, the distal end of the syringe is detached from a proximal end of the applicator tip 56, and a stylus or stick is inserted into the cannula of the applicator tip to force the residual or stranded flowable hemostat from the distal end of the applicator tip. This extra step during a surgical procedure wastes time and forces the surgeon to remove his or her eyes from the surgical site as they focus on advancing a stick through the cannula to expel the stranded flowable hemostat from the distal end of the applicator tip.

In other instances, it is difficult to express and apply high viscosity paste biologics using normal syringes due to the need to generate high expression forces. When expressing high viscosity paste biologics, in order to reduce the expression forces, it is often necessary to use cannulas having larger size lumens, which will typically generate a significant amount of paste residue (e.g., waste, or dead volume) in the cannula. In some instances, a long rod is used to empty the paste from the cannula, which results is an interruption in paste application when switching to rod insertion, and two hand operation (i.e., one hand holding the cannula and the other hand pushing the rod).

In view of the above, there remains a need for improved systems, devices, and methods for quickly, efficiently, and safely expressing flowable, therapeutic compositions during surgical procedures for controlling bleeding.

There also remains a need for improved systems, devices, and methods of efficiently and effectively expressing a flowable, therapeutic material while minimizing waste and eliminating the presence of stranded material adjacent the distal ends of cannulas and dispensing tubes.

There is also a need for improved systems, devices, and methods of expressing flowable, therapeutic compositions that make it easier for medical personnel to dispense highly viscous materials.

Moreover, there is a need for improved systems, devices, and methods of expressing flowable, therapeutic compositions that use less parts and/or components for more economically and effectively dispensing the flowable compositions.

WO 2017/079136 discloses a system for transfer of tissue such as adipose tissue. The system has a cannula with a suction and dispensing tip monolithically integrated with the cannula and having a dispensing channel in this tip. A piston having a conical distal end is provided in the cannula and a positive or negative pressure applied at the proximal end of the cannula is used to advance the piston into or out of the interior portion of the cannula. The positive and negative pressures are provided by a mechanical pump.

### SUMMARY OF THE INVENTION

The invention is defined in the appended claims. Any methods disclosed herein do not form part of the claimed invention. In one embodiment, the present patent discloses using a cannula or a hollow tube as a primary container for a flowable, therapeutic composition (e.g., a flowable hemostat; biologics; medicine paste), while using a syringe or a hand pump that contains a pumping fluid (e.g., saline) as the driving means for dispensing the flowable, therapeutic composition from a distal end of the cannula.

In one embodiment, the volume of the pumping fluid (e.g., saline) that is used is equal to the volume of the flowable, therapeutic composition that is expressed from the distal end of the cannula. Since the cannula is pre-filled with the flowable, therapeutic composition, immediately after the start of an expression procedure, the flowable, therapeutic composition exits from the distal end of the cannula. Thus, there is no waiting period or delay as the flowable, therapeutic composition flows to the distal end of the cannula.

According to the invention, a system for delivering a flowable, therapeutic composition preferably includes a cannula (e.g., a hollow tube) having a proximal end with a proximal opening, a distal end with a distal opening (e.g., a dispensing opening), and an elongated lumen extending from the proximal end to the distal end.

In one embodiment, the elongated lumen of the cannula has an inner diameter with a dimension that remains consistent (i.e., the same; unchanged) between the proximal and distal ends of the cannula.

In one embodiment, a distal end of the elongated lumen may have a constriction that enables a flowable, therapeutic composition to flow through the constriction while preventing a piston from passing through the constriction.

According to the invention, a flowable, therapeutic composition fills the elongated lumen of the cannula.

In one embodiment, the flowable, therapeutic composition may be a flowable hemostat, such as a flowable hemostat sold by Ethicon, Inc. of Somerville, New Jersey under the trademark SURGIFLO^{®} Hemostatic Matrix Kit with Thrombin.

According to the invention, a piston, also referred to herein as a cannula piston, is disposed within the elongated lumen of the cannula for sealing the proximal end of the cannula.

According to the invention, an end cap is releasably secured to the distal end of the cannula. The end cap may be unsecured from the distal end of the cannula for filling the cannula with the flowable, therapeutic composition, and the end cap may then be resecured to the distal end of the cannula for sealing the therapeutic composition within the cannula.

In one embodiment, after the cannula has been filled with the therapeutic composition, the end cap may be re-secured to the distal end of the cannula and preferably remains in place during shipment and/or storage of the delivery device. In one embodiment, the end cap is removed prior to expressing the therapeutic composition from the distal end of the cannula.

In one embodiment, the distal end of the elongated lumen of the cannula may have a constriction for preventing the piston from exiting the distal end of the cannula.

In one embodiment, an end cap may function as an applicator tip for dispensing a therapeutic composition from the distal end of the cannula of the delivery device and may have a constriction for preventing the piston from exiting the distal end of the cannula.

In one embodiment, the end cap may have an expression tip to express the flowable, therapeutic composition.

According to the invention, the system includes a pump assembly that includes a fluid and is coupled with the proximal end of the cannula. The pump assembly is operable to pump the fluid (e.g., saline) to direct the fluid to flow through the proximal opening of the cannula for forcing the piston to slide toward the distal end of the cannula to express the flowable, therapeutic composition from the distal end of the cannula.

In one embodiment, the fluid (e.g., saline) that is directed into the proximal opening at the proximal end of the cannula is used as a driving media to push the piston toward the distal end of the cannula for expressing the flowable, therapeutic composition (e.g., for flowable hemostat application).

In one embodiment, the hydraulic pressure of a fluid (e.g., saline) is used to move the piston from the proximal end to the distal end of the cannula for expressing the flowable, therapeutic composition (e.g., a flowable hemostat; biologics; medicine paste) from the distal end of the cannula, thereby providing a number of benefits including but not limited to instantaneous delivery of the flowable, therapeutic composition from the distal end of the cannula upon commencing operation of the pump assembly, minimal waste of the flowable, therapeutic composition because when the piston reaches the distal end of the cannula all of the flowable, therapeutic composition has been expelled from the cannula, less resistance to expressing the flowable, therapeutic composition due to using a pump assembly that relies upon hydraulics, and the need for fewer parts.

In one embodiment, as the piston moves distally, the piston preferably forces the flowable, therapeutic composition toward the distal end of the cannula for being expressed and/or delivered via the dispensing opening at the distal end of the cannula.

In one embodiment, the cannula may have a flexible section that enables the distal end of the cannula to be flexed into a curved configuration.

In one embodiment, the cannula may have a rigid section and a flexible section.

In one embodiment, the flowable, therapeutic composition may be a viscous fluid (e.g., a flowable hemostat; biologics), and the fluid that is pumped by the pump assembly may include saline, water, or air having a lower viscosity than the flowable, viscous fluid.

In one embodiment, the piston may have an outer perimeter that forms a seal with an inner surface of the cannula.

In one embodiment, the piston may include a cylindrical body and at least one O-ring coupled with the cylindrical body.

In one embodiment, the at least one O-ring preferably has an outer perimeter that is configured to slide over the inner surface of the cannula for forming a seal with the inner surface of the cannula.

In one embodiment, the piston that is disposed within the cannula may include a cylindrical shaped body, a flexible body, a body made of rubber, a body made of a fluoropolymer elastomer and synthetic rubber compound sold under the trademark VITON^{®}, and/or one or more balls.

In one embodiment, the pump assembly may include a syringe with a syringe barrel and a syringe plunger disposed inside the syringe barrel.

In one embodiment, the pump assembly may include a connector having a proximal end coupled with a distal end of the syringe barrel and a distal end coupled with the proximal end of the cannula.

In one embodiment, the connector has a fluid conduit that extends from the proximal end to the distal end of the connector to define a fluid flow path between the syringe barrel and the proximal end of the cannula.

In one embodiment, the syringe plunger is depressible toward the distal end of the syringe barrel to expel the fluid from the syringe barrel and force the fluid through the fluid flow path of the connector and into the elongated lumen of the cannula at the proximal end of the cannula.

In one embodiment, the pump assembly may include a hand pump such as a trigger.

In one embodiment, a specially designed hand pump may be used for delivering highly viscous compositions (e.g., medicine pastes) that requires high expressing forces.

In one embodiment, the pump assembly desirably includes a fluid reservoir for storing the fluid of the pump assembly, a pump chamber that is in fluid communication with the fluid reservoir, a plunger disposed within the pump chamber, whereby the plunger is moveable in a first direction for generating a vacuum within the pump chamber for drawing the fluid into the pump chamber and is moveable in a second direction for forcing the fluid from the pump chamber, and a squeezable trigger coupled with the plunger for moving the plunger within the pump chamber.

In one embodiment, the cannula is used as the primary container for the flowable, therapeutic composition, and a saline syringe or hand pump is used as the driving means to dispense the flowable, therapeutic composition from the cannula.

In one embodiment, using saline to drive the flowable, therapeutic composition from the cannula takes advantage of saline availability in the surgery room. The amount of the saline that is used is the same as the amount of flowable, therapeutic composition that is applied. Since the cannula is pre-filled, the flowable, therapeutic composition immediately exits the distal end of the cannula during application, and there is no waiting time for the therapeutic composition to flow from the proximal end to the distal end of the cannula.

In one embodiment, a system for delivering a flowable, therapeutic composition (e.g., a hemostatic paste) desirably includes a cannula having a proximal end with a proximal opening, a distal end with a distal opening (e.g., a dispensing opening), and an elongated lumen extending from the proximal end to the distal end.

In one embodiment, a flowable, therapeutic composition fills the elongated lumen of the cannula, and a piston is disposed within the elongated lumen. The piston preferably forms a seal with an inner surface of the cannula and is configured to slide distally within the elongated lumen of the cannula.

According to the invention, an end cap is secured to the distal end of the cannula.

According to the invention, a pump assembly is coupled with the proximal end of the cannula. The pump assembly is operable to direct a fluid (e.g., saline) to flow through the proximal opening of the cannula to force the piston toward the distal end of the cannula for expressing the flowable, therapeutic composition from the distal end of the cannula.

In one embodiment, the seal formed by the piston isolates the pumping fluid (e.g., saline) from the flowable, therapeutic composition (e.g., hemostatic paste). The pumping fluid may be in contact with a proximal side of the piston and the flowable, therapeutic composition may be in contact with the distal side of the piston.

In one embodiment, the pump assembly may include a syringe including a syringe barrel and a syringe plunger that is disposed inside the syringe barrel with the saline filling the syringe barrel.

In one embodiment, the pump assembly may include a connector having a proximal end coupled with a distal end of the syringe barrel and a distal end coupled with the proximal end of the cannula.

In one embodiment, the connector has a fluid conduit that extends from the proximal end to the distal end of the connector to define a fluid flow path for the saline between the syringe barrel and the proximal end of the cannula.

In one embodiment, the syringe plunger is depressible toward the distal end of the syringe barrel to expel the saline from the syringe barrel and force the saline through the fluid flow path of the connector and into the proximal end of the cannula for contacting a proximal side of the piston.

In one embodiment, the pump assembly may include a fluid reservoir for storing the saline, a pump chamber that is in fluid communication with the fluid reservoir, and a plunger disposed within the pump chamber.

In one embodiment, the plunger is moveable in a first direction for generating a vacuum for drawing the saline from the fluid reservoir and into the pump chamber and is moveable in a second direction for forcing the saline from the pump chamber.

In one embodiment, a squeezable trigger is coupled with the plunger for moving the plunger within the pump chamber.

In one embodiment, prior to dispensing or expressing a flowable, therapeutic composition, the end cap may be removed from the distal end of the cannula so that the flowable, therapeutic composition may flow through an opening at the distal end of the cannula.

In one embodiment, a method of delivering a therapeutic composition preferably includes obtaining a cannula having a proximal end, a distal end, and an elongated lumen extending from the proximal end to the distal end. The elongated lumen of the cannula is filled with a flowable, therapeutic composition, a piston is disposed within the elongated lumen of the cannula, and an end cap is connected with the distal end of the cannula.

In one embodiment, the method includes directing a fluid into the elongated lumen at the proximal end of the cannula to force the piston to slide within the elongated lumen of the cannula and toward the distal end of the cannula to express the flowable, therapeutic composition from the distal end of the cannula, such as through a dispensing opening located at the distal end of the cannula.

In one embodiment, the method may include different expression stages including a first expression stage during which the piston is located at the proximal end of the cannula for sealing a proximal opening at the proximal end of the cannula with the flowable, therapeutic composition extending between a distal side of the piston and the distal end of the cannula.

In one embodiment, the method may include a second expression stage during which the piston is located midway between the proximal and distal ends of the cannula with the fluid extending between a proximal side of the piston and the proximal end of the cannula and the flowable, therapeutic composition extending between a distal side of the piston and the distal end of the cannula.

In one embodiment, the method may include a third expression stage during which the piston is located at the distal end of the cannula with the fluid extending between the proximal side of the piston and the proximal end of the cannula.

In one embodiment, the step of directing the fluid into the elongated lumen at the proximal end of the cannula may include coupling a pump assembly with the proximal end of the cannula and operating the pump assembly to force the fluid into the elongated lumen at the proximal end of the cannula.

High viscosity paste biologics are difficult to apply when using normal syringes due to the requirement to generate high expression forces. One solution is to use cannulas having large sized lumens to reduce the level of expression force that is required. This solution will generate a significant amount of residue (e.g., waste, or dead volume) in the cannula. In some instances, a long rod is used to empty contents of the cannula, resulting in an interruption of the paste application by switching to rod insertion, and two hand operation (one hand holding the cannula, the other hand inserting and pushing the rod).

In one embodiment, the cannula is the primary container for the flowable, therapeutic composition (e.g., medicine paste), while a syringe or hand pump that contains the fluid (e.g., saline) is used as the driving means to dispense the flowable, therapeutic composition from the distal end of the cannula, taking advantage of saline availability in an operating room. The amount of fluid (e.g., saline) that is used is the same as the amount of flowable, therapeutic composition (e.g., flowable hemostat; medicine paste) that is expressed. Since the cannula is pre-filled with the flowable, therapeutic composition, the flowable, viscous material immediately exits the distal end of the cannula after activation of the driving means. Thus, there is no waiting time for the flowable, therapeutic composition to flow from the proximal end to the distal end of the cannula.

In embodiments in which the cannula has a flexible section that can be bent and/or curved, the piston may be ball shaped. In one embodiment, the piston preferably includes a series of balls (e.g., three balls) to accommodate the curved shape of the flexible section of the cannula with no obstruction of piston movement.

In one embodiment, for therapeutic compositions having a low viscosity, air may be used as the driving media instead of saline or water.

In one embodiment, the piston may be shaped in a way that leaves no residue within the cannula (i.e., all the flowable, therapeutic composition in the cannula is dispensed and no flowable, therapeutic composition is left in the cannula).

In one embodiment, the cannula may be either pre-filled with the flowable, therapeutic composition, or be filled on-site at the time of a surgical procedure.

In one embodiment, a system for delivering a flowable, therapeutic composition may include a dual lumen cannula with a first component of the flowable, therapeutic composition disposed within a first lumen and a second component of the flowable, therapeutic composition disposed within a second lumen and a distinct syringe/pump assembly being coupled with each respective lumen.

For the dual lumen embodiment disclosed above, the sizes of the respective syringes may control the ratio of the two components that are mixed together to form the flowable, therapeutic composition.

### BRIEF DESCRIPTION OF THE DRAWINGS

FIG. 1A is a perspective view of system for delivering a flowable, therapeutic composition, in accordance with one embodiment of the present patent.
FIG. 1B is another perspective view of the system for delivering a flowable, therapeutic composition shown in FIG. 1A.
FIG. 2 is an exploded view of the system for delivering a flowable, therapeutic composition shown in FIGS. 1A and 1B.
FIG. 3 is an exploded view of a pump assembly of the system for delivering a flowable, therapeutic composition shown in FIGS. 1A, 1B and 2.
FIG. 4 is an exploded view of a distal end of the system for delivering a flowable, therapeutic composition shown in FIGS. 1A, 1B and 2.
FIG. 5A is a cross-sectional view of a system for delivering a flowable, therapeutic composition during a first expression stage with a pump assembly located in a retracted position, in accordance with one embodiment of the present patent application.
FIG. 5B is a cross-sectional view of the system shown in FIG. 5A during a second expression stage with the pump assembly in an intermediate position, in accordance with one embodiment of the present patent application.
FIG. 5C is a cross-sectional view of the system shown in FIGS. 5A and 5B during a third expression stage with the pump assembly in an extended position, in accordance with one embodiment of the present patent application.
FIG. 6 is a magnified view of the pump assembly of the system shown in FIG. 5B during the second expression stage.
FIG. 7 is a magnified view of a cannula and a piston during the second expression stage shown in FIG. 5B.
FIG. 8A is a perspective view of a cannula piston of a system for delivering a flowable, therapeutic composition, in accordance with one embodiment of the present patent application.
FIG. 8B is a side elevation view of the cannula piston shown in FIG. 8A.
FIG. 9 is a side elevation view of the cannula piston shown in FIG. 8B with first and second O-rings assembled with a proximal section of the cannula piston, in accordance with one embodiment of the present patent application.
FIG. 10 is a cross-sectional view of a cannula of a system for delivering a flowable, therapeutic composition, and a cannula piston disposed within an elongated lumen of the cannula, in accordance with one embodiment of the present patent applications.
FIG. 11 is a cross-sectional view of a distal end of a system for delivering a flowable, therapeutic composition including a cannula having an elongated lumen with a cannula piston disposed within the elongated lumen of the cannula, in accordance with one embodiment of the present patent application.
FIG. 12 is a cross-sectional view of a distal end of a system for delivering a flowable, therapeutic composition including a cannula having an elongated lumen, a cannula piston disposed within the elongated lumen, and an end cap secured to a distal end of the cannula, in accordance with one embodiment of the present patent application.
FIG. 13A is a side view of a cannula of a system for delivering a flowable, therapeutic composition, the cannula including a rigid section and a flexible section, in accordance with one embodiment of the present patent application.
FIG. 13B is a side view of the cannula shown in FIG. 13A with the flexible section of the cannula bent into a curved configuration.
FIG. 14A shows the cannula of FIG. 13B with a piston disposed within an elongated lumen of the curved flexible section of the cannula, the piston including a series of balls disposed within the elongated lumen of the cannula, in accordance with one embodiment of the present patent application.
FIG. 14B is a magnified view of the cannula shown in FIG. 14A with the cannula piston including the series of balls disposed within a curved portion of the flexible cannula section, in accordance with one embodiment of the present patent application.
FIG. 15 is a system for delivering a flowable, therapeutic composition including a first cannula that contains a first part of the flowable, therapeutic composition and a second cannula that contains a second part of the flowable, therapeutic composition, in accordance with one embodiment of the present patent application.
FIG. 16A is a perspective view of a distal end of a system for delivering a flowable, therapeutic composition, the system including a trigger that is squeezable for activating a pump for expressing the flowable, therapeutic composition, in accordance with one embodiment of the present patent application.
FIG. 16B is a perspective view of a proximal end of the system for delivering a flowable, therapeutic composition shown in FIG. 16A.
FIG. 17 is a cross-sectional view of the system for delivering a flowable, therapeutic composition shown in FIGS. 16A and 16B.
FIG. 18 is a cross-sectional view of a pump assembly of the system for delivering a flowable, therapeutic composition shown in FIG. 17.
FIG. 19 is a cross-sectional view of a proximal end of a cannula with a cannula piston disposed within an elongated lumen of the cannula of the system for delivering a flowable, therapeutic composition shown in FIG. 17.
FIG. 20 shows a prior art device for dispensing a flowable hemostat.
FIG. 21A shows a first stage of a prior art method for dispensing a flowable hemostat to stop bleeding at a surgical site.
FIG. 21B shows a second stage of a prior art method for dispensing a flowable hemostat to stop bleeding at a surgical site.

### DETAILED DESCRIPTION OF PREFERRED EMBODIMENTS

### Definitions.

"Biocompatible" refers to a material's ability to perform its intended function without eliciting any substantial undesirable local or systemic effects in the host.

"Biologics" are biological products such as vaccines, blood, and blood components, allergenics, somatic cells, gene therapy, tissues, and recombinant therapeutic proteins. Biologics can be composed of sugars, proteins, or nucleic acids or complex combinations of these substances, or may be living entities such as cells and tissues. Biologics are isolated from a variety of natural sources - human, animal, or microorganism - and may be produced by biotechnology methods and other cutting-edge technologies. Gene-based and cellular biologics, for example, often are at the forefront of biomedical research, and may be used to treat a variety of medical conditions for which no other treatments are available. See https://www.fda.gov/about-fda/center-biologics-evaluation-and-research-cber/what-are-biologics-questions-and-answers.

A "flowable hemostat" is a composition that is designed to actively help in blood clotting. Flowable hemostats speed up the conversion of a substance in the blood called fibrin, to fibrinogen. This chemical reaction leads to the formation of blood clots, which is the final step in the process of hemostasis. Flowable hemostats are made of a mostly liquid mixture of biomaterials like gelatin and cellulose mixed with thrombin. An example of a flowable hemostat is sold by Ethicon, Inc. of Somerville, New Jersey under the trademark SURGIFLO^{®} Hemostat Matrix Kit with Thrombin.

"Hemostasis" is a process which causes bleeding to diminish or stop. Hemostasis occurs when blood is present outside of the body or blood vessels and is the instinctive response for the body to stop bleeding and loss of blood. During hemostasis three steps occur in a rapid sequence. Vascular spasm is the first response as the blood vessels constrict to allow less blood to be lost. In the second step, platelet plug formation, platelets stick together to form a temporary seal to cover the break in the vessel wall. The third and last step is called coagulation or blood clotting. Coagulation reinforces the platelet plug with fibrin threads that act as a "molecular glue." A hemostatic compound, such as the flowable hemostat sold by Ethicon, Inc. of Somerville, New Jersey under the trademark SURGIFLO^{®} Hemostat Matrix Kit with Thrombin, is capable of stimulating hemostasis.

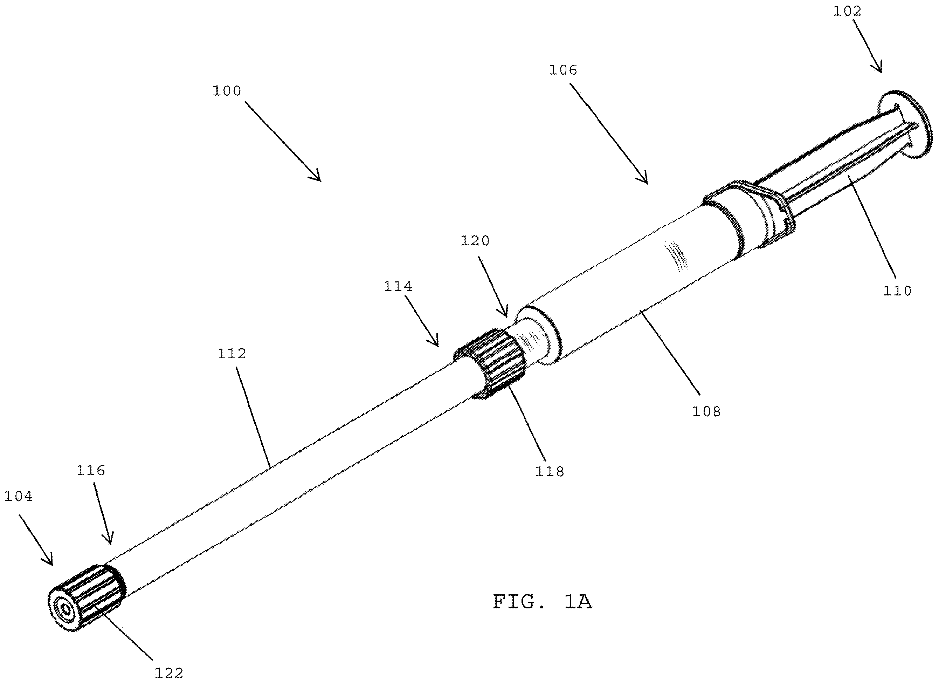

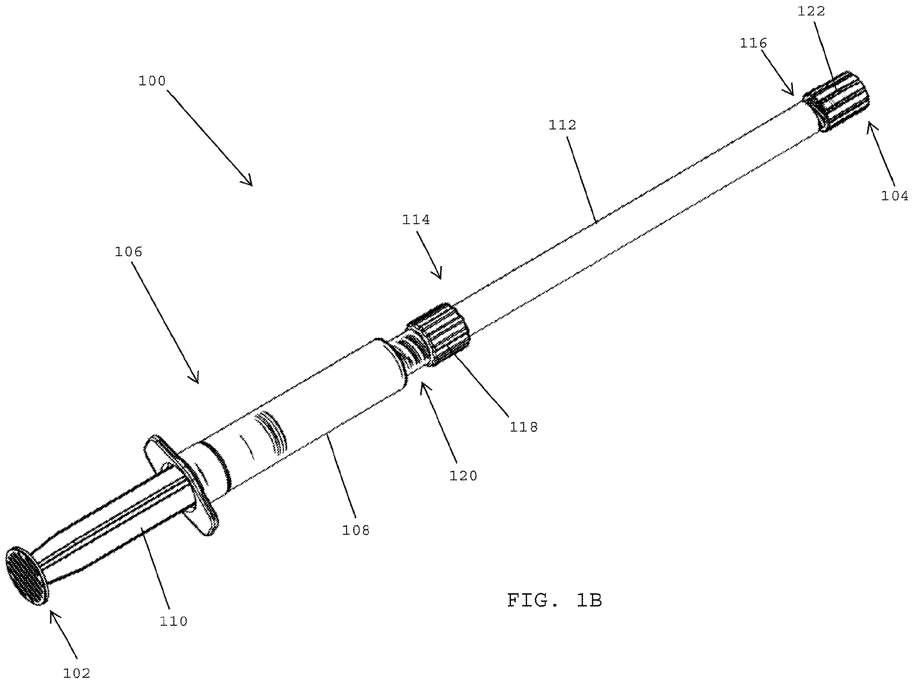

Referring to FIGS. 1A and 1B, in one embodiment, a system for delivering a flowable, therapeutic composition, such as the flowable hemostat sold by Ethicon, Inc. of Somerville, New Jersey under the trademark SURGIFLO^{®} Hemostat Matrix Kit with Thrombin, biologics, and medicine paste, preferably includes a delivery device 100 having proximal end 102 and a distal end 104. In one embodiment, the delivery device 100 is preferably made of biocompatible materials. In one embodiment, the delivery device 100 preferably includes a pump assembly 106 (also referred to as a pump) comprising a syringe barrel 108 and a syringe plunger 110.

In one embodiment, the delivery device 100 preferably includes a cannula 112 (e.g., a hollow tube) having a proximal end 114 and a distal end 116. The delivery device 100 preferably includes a connector 118 that is utilized for securing a distal end 120 of the syringe barrel 108 with the proximal end 114 of the cannula 112.

According to the invention, the distal end 104 of the delivery device 100 includes an end cap 122 that is secured to the distal end 116 of the cannula 112. According to the invention, the end cap 122 is releasably secured to the distal end 116 of the cannula 112. The end cap 122 may be removed for uncovering an opening (e.g., a dispensing opening) located at the distal end 116 of the cannula 112 so that the cannula may be filled with a flowable, therapeutic composition (e.g., a flowable hemostat; biologics; medicine paste).

According to the invention, the flowable, therapeutic composition is disposed within the cannula. In one embodiment, activating the pump assembly 106 (FIG. 1A) preferably expels the flowable, therapeutic composition from the distal end of the cannula.

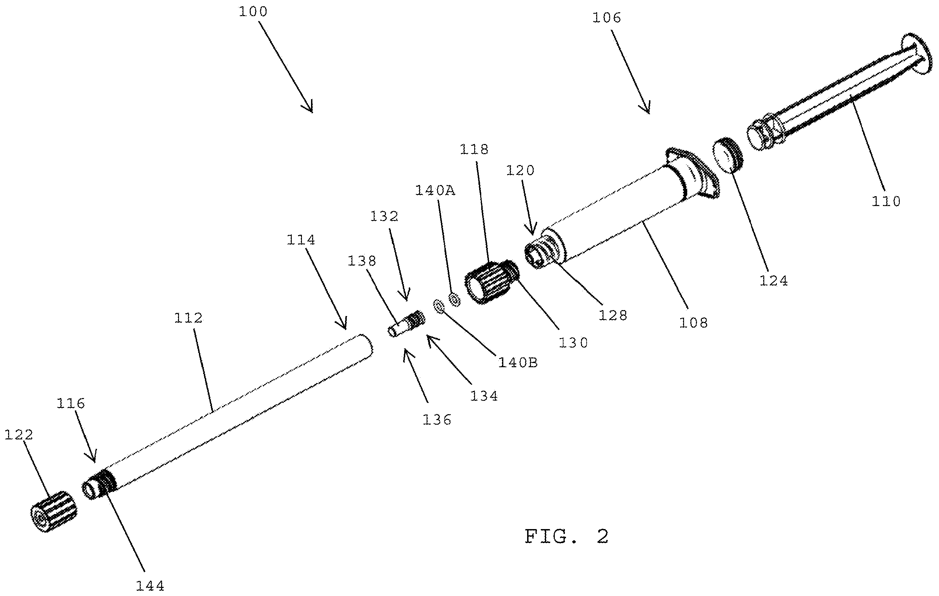

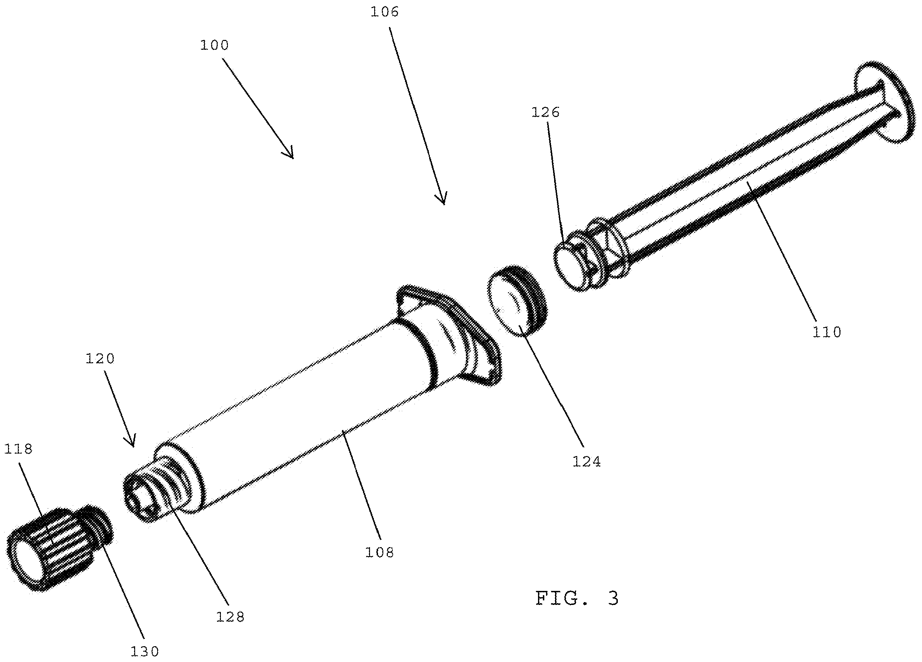

Referring to FIGS. 2 and 3, in one embodiment, the delivery device 100 preferably includes the pump assembly 106 comprising the syringe barrel 108 and the syringe plunger 110. The syringe plunger 110 is insertable into an opening at the proximal end of the syringe barrel 108. A syringe plunger piston 124 is secured to the distal end 126 of the syringe plunger 110.

The distal end 120 of the syringe barrel 108 has internal threads 128 that are adapted to receive external threads 130 provided at a proximal end of the connector 118. The connector enables a fluid-tight connection to be formed between the distal end 120 of the syringe barrel 108 and the proximal end 114 of the cannula 112.

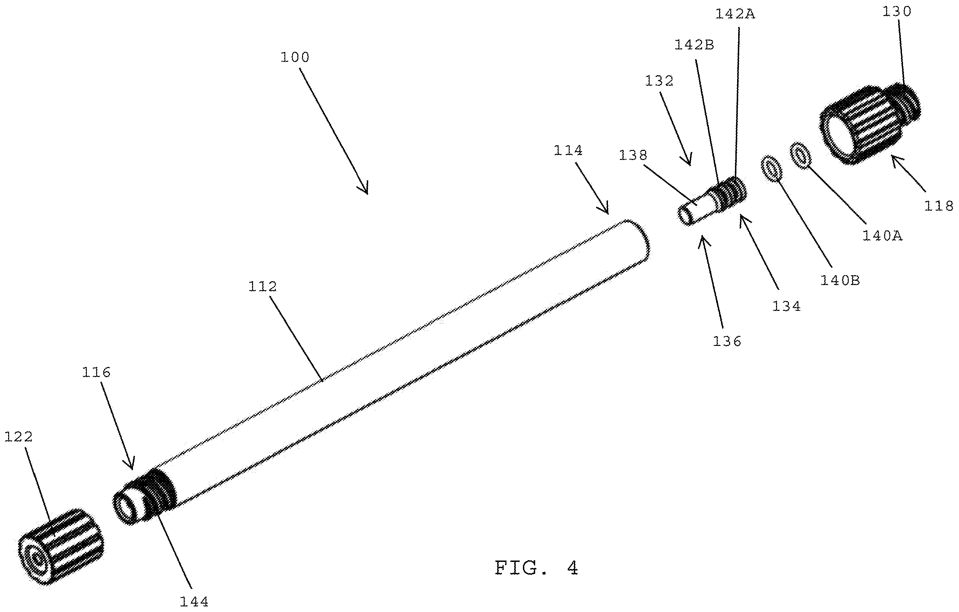

Referring to FIGS. 2 and 4, in one embodiment, the delivery device 100 preferably includes a cannula piston 132 that is disposed within an elongated lumen of the cannula 112. The cannula piston 132 is preferably adapted for forcing the flowable, therapeutic composition toward the distal end 116 of the cannula 112. According to the invention, the cannula piston 132 includes a proximal section 134 and a distal section 136 having a distally extending nose 138.

The delivery device 100 preferably includes first and second O-rings 140A, 140B that are adapted to be secured over recesses 142A, 142B located within the proximal section 134 of the cannula piston 132. The first and second O-rings 140A, 140B preferably have outer perimeters that form a seal with an inner surface of the cannula 112.

According to the invention, the cannula piston 132 is located within an elongated lumen of the cannula 112. In one embodiment, at the beginning of an expression procedure, the cannula piston 132 including the O-rings 140A, 140B are initially disposed at the proximal end 114 of the cannula 112, and the subassembly of the cannula 112 and the piston 132 is assembled with the connector 118 for facilitating linking and/or coupling the cannula 112 with the syringe barrel 108.

In one embodiment, the distal end 116 of the cannula 112 preferably includes external threads 144 that are adapted to mesh with internal threads (not shown) provided on the end cap 122. The external threads 144 at the distal end 116 of the cannula 112 and the internal threads on the end cap 122 preferably enable the end cap 122 to be releasably secured to the distal end 116 of the cannula 112. The end cap 122 may be disconnected (e.g., unscrewed) from the distal end 116 of the cannula 112 for filling the cannula with a flowable, therapeutic composition. In one embodiment, after filling the cannula with the flowable, therapeutic composition, the end cap may be re-secured to the distal end of the cannula.

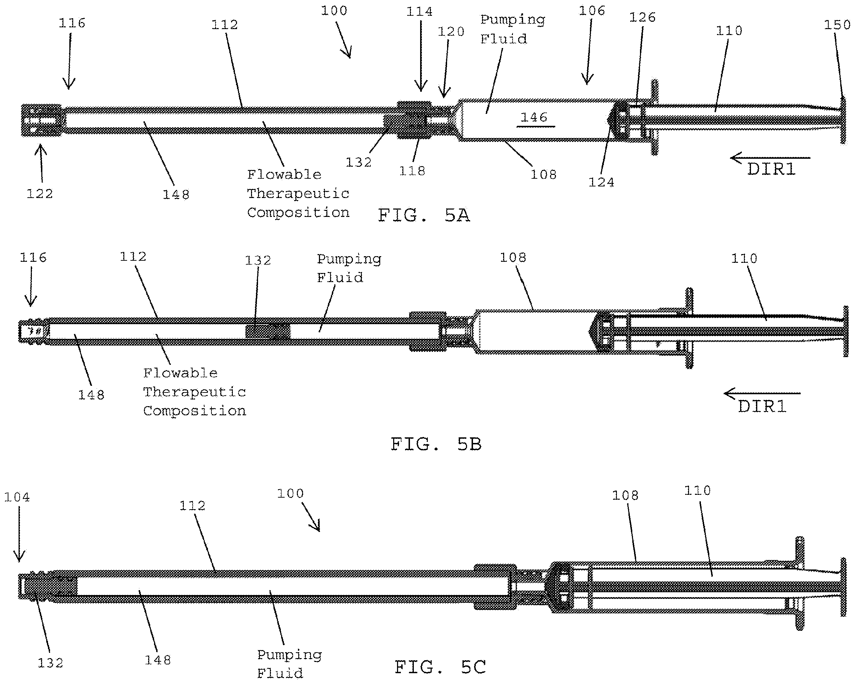

Referring to FIG. 5A, in one embodiment, the syringe plunger piston 124 may be secured to the distal end 126 of the syringe plunger, and the distal end 126 of the syringe plunger 110 may be inserted into an opening at the proximal end of the syringe barrel 108. In one embodiment, the outer perimeter of the syringe plunger piston 124 and an inner surface of the syringe barrel 108 preferably form a fluid-tight seal therebetween. In one embodiment, the syringe barrel 108 includes a syringe barrel chamber 146 (i.e., a pump chamber) that is adapted to be filled with a pumping fluid such as saline, water, or air.

In one embodiment, the proximal end 114 of the cannula 112 is secured to the distal end 120 of the syringe barrel 108 by threading the external threads 130 (FIG. 3) of the connector 118 into the internal threads 128 (FIG. 3) located at the distal end 120 of the syringe barrel 108.

According to the invention, the cannula 112 includes an elongated lumen 148 that extends from the proximal end 114 to the distal end 116 of the cannula 112. In one embodiment, the elongated lumen 148 of the cannula 112 may be filled with a flowable, therapeutic composition (e.g., a hemostatic paste) by unthreading the end cap 122 from the distal end 116 of the cannula 112. The cannula may be filled with the flowable, therapeutic composition at a manufacturing facility or within a surgical environment. After the elongated lumen 148 of the cannula 112 has been filled with the flowable, therapeutic composition, the end cap 122 may be re-secured to (e.g., threaded onto) the distal end 116 of the cannula 112.

In one embodiment, prior to dispensing the flowable, therapeutic composition from the distal end 116 of the cannula 112, the cannula piston 132 is located at the proximal end 114 of the cannula 112 (FIG. 5A). In one embodiment, the syringe barrel chamber 146 is filled with a pumping fluid (e.g., saline; air) and the elongated lumen 148 of the cannula 112 is filled with a flowable, therapeutic composition. The cannula piston 132 preferably forms a seal with the inner surface of the cannula 112 and isolates the saline disposed within the syringe barrel chamber 146 from the flowable, therapeutic composition disposed within the elongated lumen 148 of the cannula 112.

FIG. 5A shows the end cap 122 secured to the distal end 116 of the cannula 112. In one embodiment, the end cap 122 remains secured to the distal end of the cannula 112 after the cannula has been filled with a therapeutic composition and during shipment and storage. During a surgical procedure, the end cap 122 is preferably removed (e.g., unscrewed) from the distal end 116 of the cannula 112 so that the therapeutic composition may be dispensed from a dispensing opening at the distal end 116 of the cannula 112.

In one embodiment, the pump assembly 106 of the flowable, therapeutic composition delivery device 100 may be activated for forcing the saline from the distal end 120 of the syringe barrel 108 which, in turn, forces the piston 132 to slide within the elongated lumen 148 toward the distal end 116 of the cannula 112. As the piston 132 is forced in the distal direction designated DIR1, the flowable, therapeutic composition within the elongated lumen 148 is dispensed via the dispensing opening at the distal end 116 of the cannula 112.

FIG. 5A shows an initial position (i.e., a first expression stage), prior to dispensing the flowable, therapeutic composition, wherein the plunger 110 is in a retracted position so that the syringe plunger piston 124 is located adjacent a proximal end of the syringe barrel 108. In one embodiment, saline is loaded into the syringe barrel chamber 146 and the flowable, therapeutic composition is disposed within the elongated lumen 148 of the cannula 112.

Referring to FIGS. 5A and 5B, in an embodiment not forming part of the claimed invention, prior to expressing the therapeutic composition, the end cap 122 (FIG. 5A) is removed for uncovering the dispensing opening at the distal end 116 of the cannula 112. In one embodiment, after the end cap is removed, in order to commence a dispensing operation, a thumb tab 150 secured to a proximal end of the syringe plunger 110 is depressed in the distal direction designated DIR1. As the syringe plunger 110 is advanced in the distal direction DIR1, the syringe plunger piston 124 moves distally within the syringe barrel chamber 146 (i.e., pump chamber) of the syringe barrel 108. The distally moving syringe plunger piston 124 forces the saline within the syringe barrel chamber 146 from the opening at the distal end 120 of the syringe barrel 108. The saline, under positive pressure, forces the cannula piston 132 to move in the distal direction DIR1 toward the distal end 116 of the cannula. The distally moving piston 132 forces the flowable, therapeutic composition (e.g., a flowable hemostat; a hemostatic paste) that fills the elongated lumen 148 to be dispensed via the dispensing opening at the distal end 116 of the cannula 112.

FIG. 5A shows the location of the cannula piston 132 at the beginning of a flowable, therapeutic composition dispensing operation. FIG. 5B shows the location of the cannula piston 132 during an intermediate stage (i.e., a second expression stage) of a flowable, therapeutic composition dispensing operation. FIG. 5C shows the location of the cannula piston 132 when the syringe plunger 110 has been fully depressed within the syringe barrel 108 and when all of the flowable, therapeutic composition within the elongated lumen 148 has been dispensed from the distal end 104 of the delivery device 100 (i.e., a third expression stage).

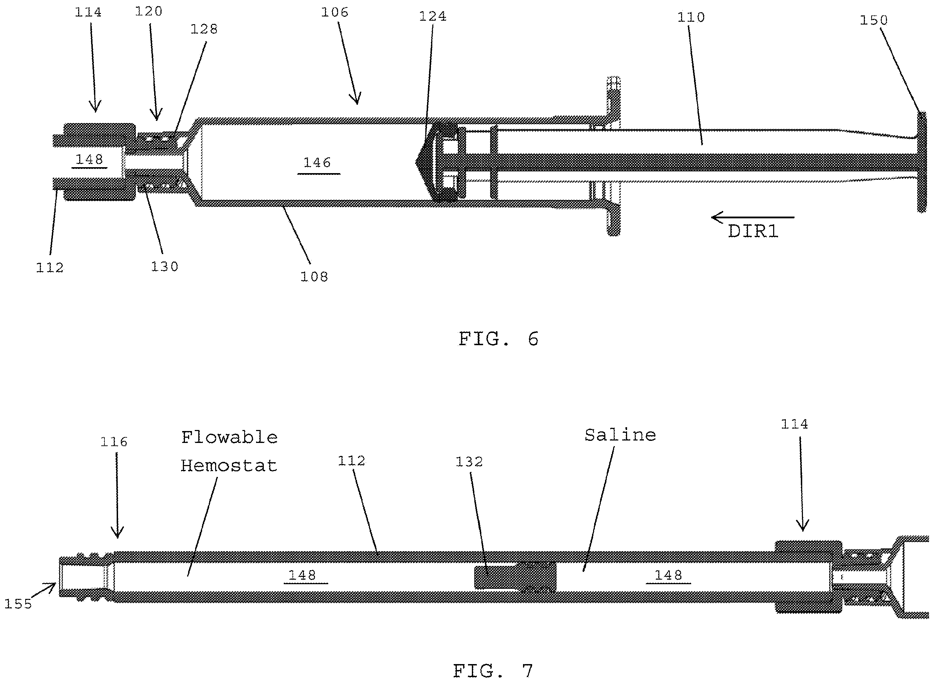

Referring to FIG. 6, in one embodiment, the pump assembly 106 of the delivery device 100 preferably includes the syringe barrel 108 and the syringe plunger 110. The syringe plunger piston 124 is secured to the distal end of the syringe plunger 110 and forms a fluid-tight seal with an inner surface of the syringe barrel 108. The distal end 120 of the syringe barrel 108 is secured to the proximal end 114 of the cannula 112 by the connector 118. The connector 118 has external threads 130 that mesh with internal threads 128 provided at the distal end 120 of the syringe barrel 108. The syringe barrel 108 includes the syringe barrel chamber 146 that is adapted to receive a pumping fluid such as saline, water, or air. In one embodiment, the distal end of the syringe barrel 108 has external threads and the proximal end of the connector 118 has internal threads that mesh with the external threads at the distal end of the syringe barrel.

The pump assembly 106 may be activated by depressing the thumb tab 150 secured to the proximal end of the syringe plunger 110 in the distal direction DIR1, which forces the saline within the syringe barrel chamber 146 from a dispensing opening at the distal end 120 of the syringe barrel 108. The distally flowing saline is directed into the elongated lumen 148 at the proximal end 114 of the cannula 112 to apply a force on the cannula piston to drive the cannula piston to the distal end of the cannula.

Referring to FIG. 7, in one embodiment, the saline under positive pressure flows into the elongated lumen 148 at the proximal end 114 of the cannula 112 for forcing the cannula piston 132 to move (e.g., slide) distally in the direction DIR1 through the elongated lumen 148 of the cannula 112. As the cannula piston 132 moves toward the distal end of the cannula, the cannula piston forces the flowable, therapeutic composition that fills the elongated lumen 148 of the cannula to be dispensed via the dispensing opening 155 located at the distal end 116 of the cannula 112.

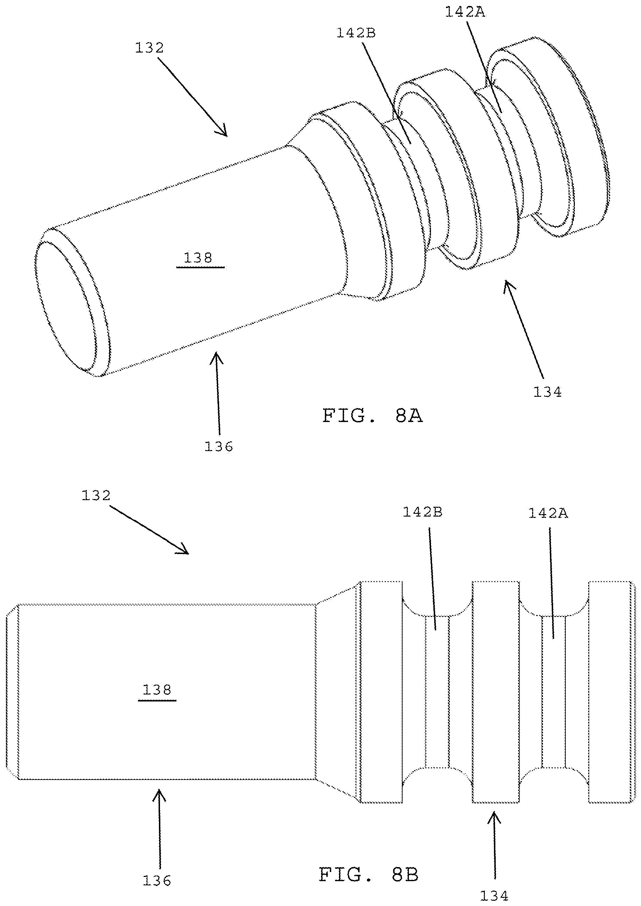

Referring to FIGS 8A and 8B, in one embodiment, the cannula piston 132 includes a proximal section 134 and a distal section 136 having a distally projecting nose 138. The proximal section 134 of the cannula piston 132 includes first and second annular depressions 142A, 142B that are adapted to seat the first and second O-rings 140A, 140B (FIG. 4).

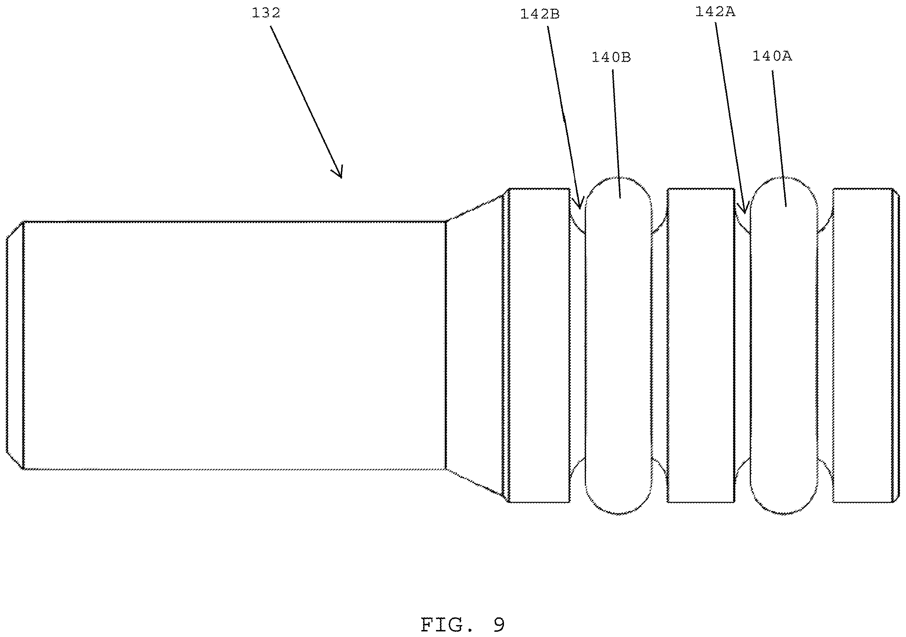

Referring to FIG. 9, in one embodiment, the cannula piston 132 preferably includes the first O-ring 140A that is secured over the first annular depression 142A and the second O-ring 140B that is secured over the second annular depression 142B. In one embodiment, the first and second O-rings 140A, 140B have outer perimeters that are adapted to engage an inner surface of the cannula 112 (FIG. 4) for forming a seal between the cannula piston 132 and the inner surface of the cannula 112 (FIG. 4).

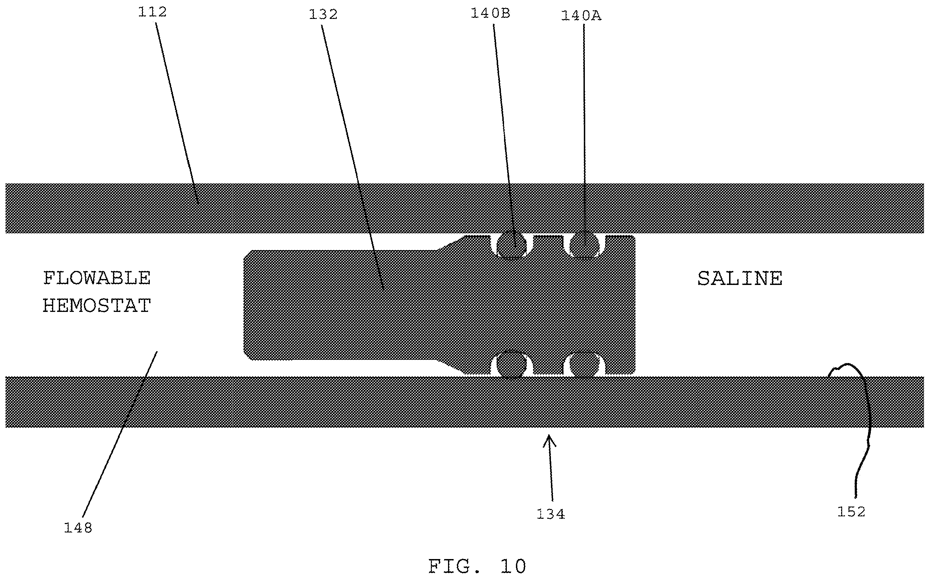

Referring to FIG. 10, in one embodiment, the cannula piston 132 is preferably disposed within the elongated lumen 148 of the cannula 112. The first and second O-rings 140A, 140B are secured with the proximal section 134 of the cannula piston 132 for forming a seal with the inner surface 152 of the cannula 112. The seal formed by the first and second O-rings 140A, 140B preferably isolates the saline that is located on the upstream side of the cannula piston 132 from the flowable, therapeutic composition that is located on the downstream side of the cannula piston 132.

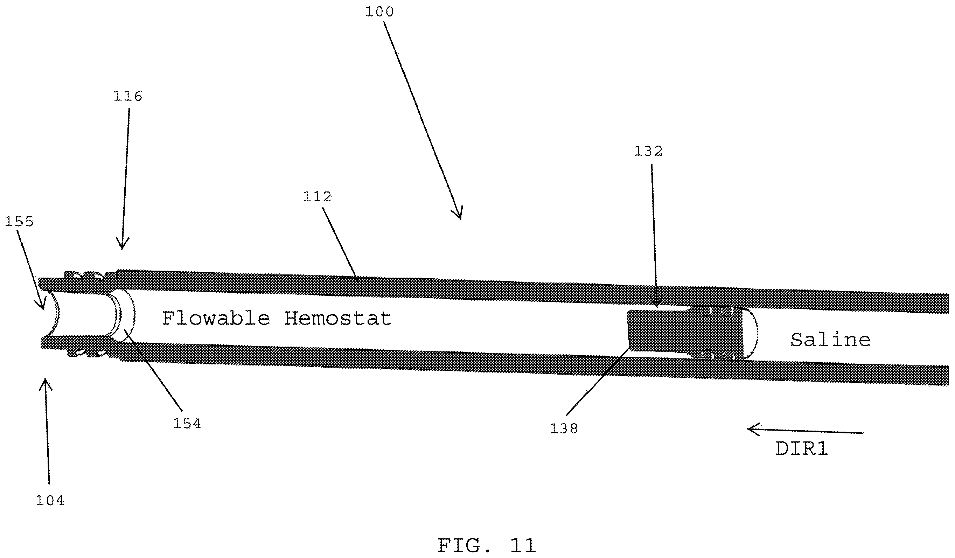

Referring to FIG. 11, in one embodiment, the cannula piston 132 divides and/or isolates the pumping fluid (e.g., saline) that is located on the upstream side of the cannula piston from the flowable, therapeutic composition (e.g., hemostatic paste) that is located on the downstream side of the cannula piston. As the saline forces the cannula piston 132 to move in the distal direction DIR1 toward the distal end 116 of the cannula 112, the cannula piston 132 forces the flowable, therapeutic composition that fills the cannula to be dispensed and/or expressed from the dispensing opening 155 located at the distal end 116 of the cannula 112. In one embodiment, in response to the hydraulic forces provided by the saline, the cannula piston 132 continues to move (e.g., slide) in a distal direction DIR1 until the nose 138 of the cannula piston 132 abuts against an internal shoulder 154 located at the distal end 116 of the cannula 112. The shoulder 154 preferably functions as a stop that prevents the cannula piston 132 from being expelled from, discharged from, and/or exiting from the distal end 104 of the delivery device 100.

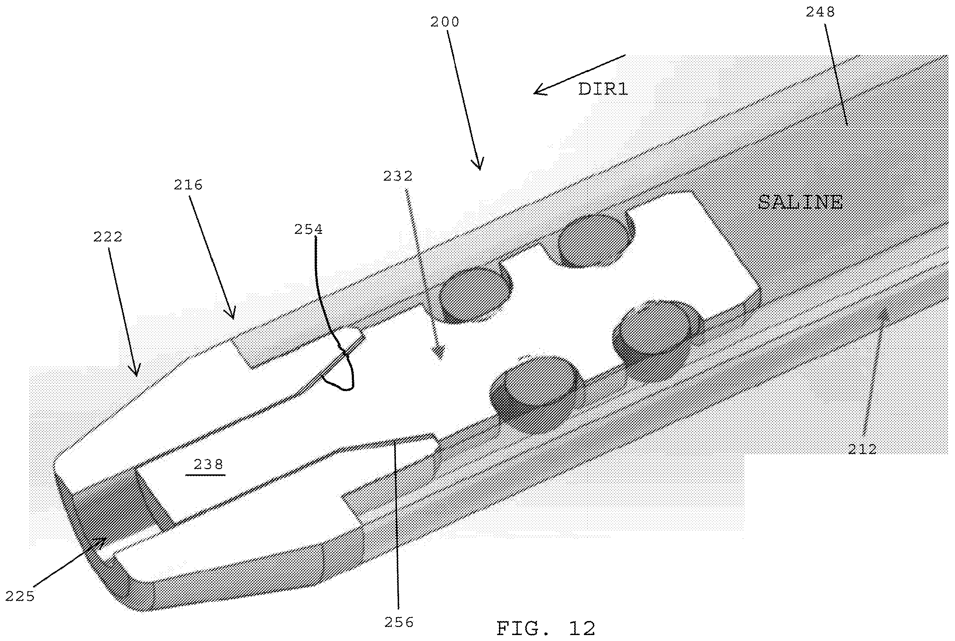

Referring to FIG. 12, in one embodiment, a flowable, therapeutic composition delivery device 200 preferably includes one or more of the features shown and described above in FIGS. 1A-11. In one embodiment, the delivery device 200 preferably includes an applicator tip 222 secured to a distal end 216 of a cannula 212. The applicator tip 222 desirably includes a proximal shoulder 254 that is adapted to engage a distal shoulder 256 of a cannula piston 232. The applicator tip 222 also preferably includes a dispensing channel 225 that is adapted to receive a distal nose 238 of the cannula piston 232. In one embodiment, as the saline that is directed into the proximal end of the elongated lumen 248 of the cannula 212 forces the cannula piston 232 to move in a distal direction DIR1, the downstream side of the cannula piston 232 forces the flowable, therapeutic composition to be dispensed via the dispensing opening 225 of the applicator tip 222. The cannula piston 232 preferably continues to move distally until the shoulder 256 on the distal side of the cannula piston 232 engages the proximal shoulder 254 of the applicator tip 222. Thus, the proximal shoulder 254 of the applicator tip 222 may function as a stop to prevent the canula piston 232 from exiting from the distal end of the cannula 212.

In one embodiment, the cannula piston 232 may be designed to fit inside the contour (e.g., a constriction) of the applicator tip to leave none or minimal residue of the flowable, therapeutic composition after an application procedure has been completed. In the distal-most position shown in FIG. 12, the nose 238 at the distal end of the cannula piston 232 is preferably positioned within the dispensing opening 225 of the applicator tip 222, which ensures that all or virtually all of the flowable, therapeutic composition disposed within the elongated lumen 248 of the cannula 212 has been dispensed, thereby minimizing waste, and ensuring that no flowable, therapeutic composition remains within the cannula 212 of the delivery device 200.

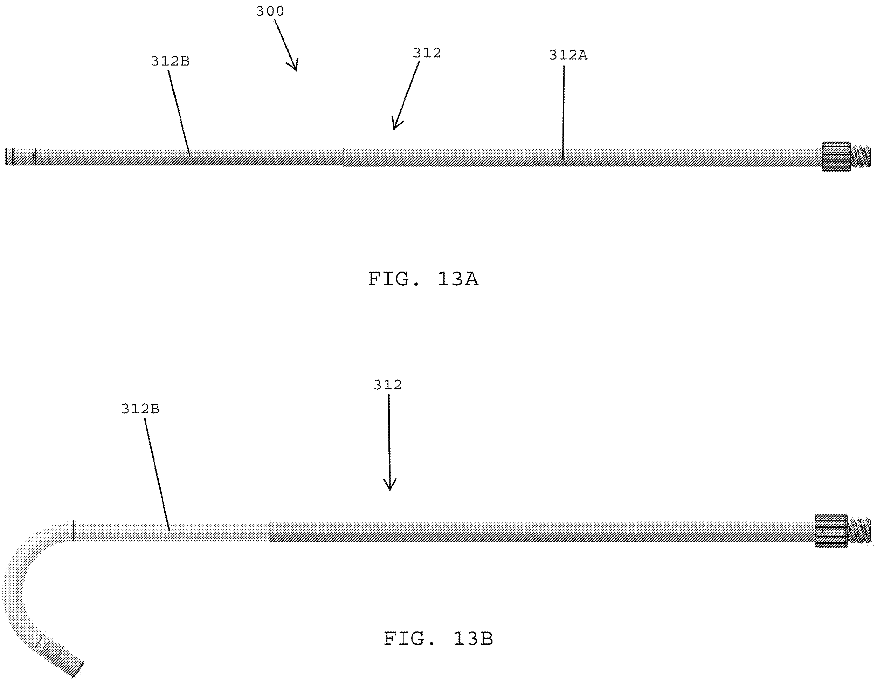

Referring to FIG. 13A, in one embodiment, a delivery device 300 preferably includes one or more of the features shown and described above in FIGS. 1A-11. In one embodiment, the delivery device 300 preferably includes a cannula 312 having a rigid section 312A and a flexible section 312B.

Referring to FIG. 13B, in one embodiment, the flexible section 312B of the cannula 312 may be flexed into a bent configuration for facilitating dispensing a flowable, therapeutic composition onto tissue of a patient.

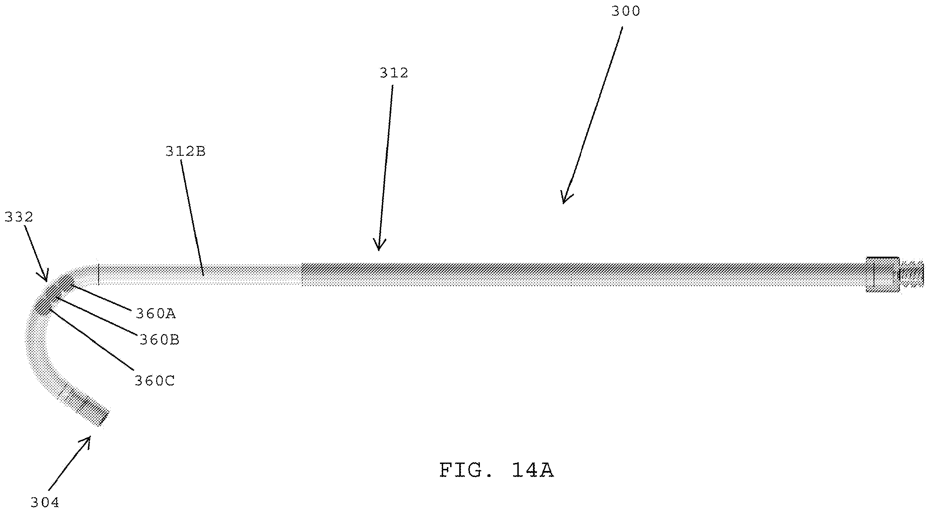

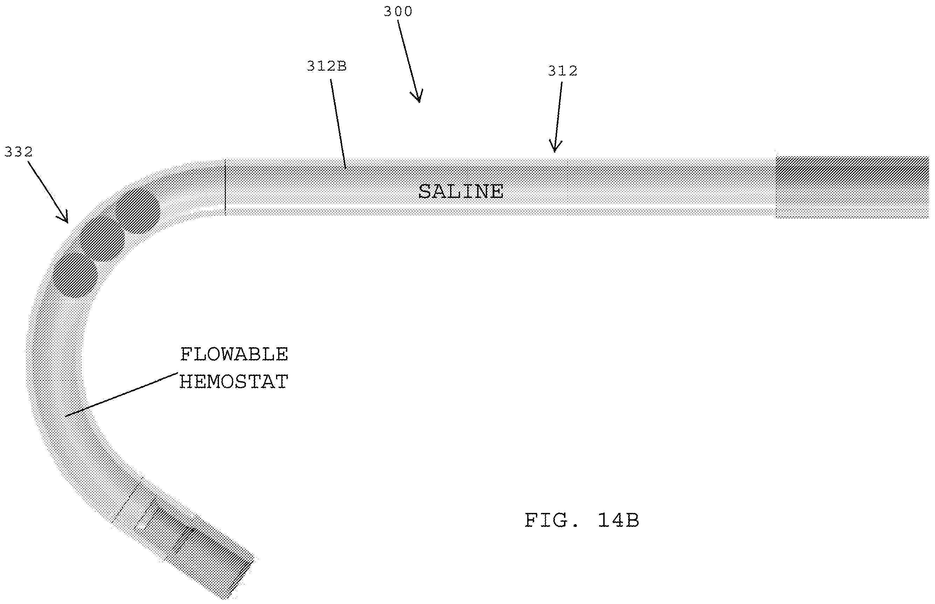

Referring to FIGS. 14A and 14B, in one embodiment, the delivery device 300 includes a cannula piston 332 that may include one or more balls 360A-360C that are able to form a seal within the flexible section 312B of the cannula 312 as the cannula piston 332 moves toward the distal end 304 of the delivery device 300.

In one embodiment, the three balls 360A-360C of the cannula piston 332 form a seal that isolates the saline on the upstream side of the cannula piston 332 from the flowable, therapeutic composition located on the downstream side of the cannula piston 332. The saline preferably forces the balls 360A-360C of the cannula piston 332 to move toward the distal end 304 of the cannula 312. The balls 360A-360C may be located within and/or pass through a curved portion of the cannula while maintaining a seal. As the cannula piston 332 moves toward the distal end of the cannula, the cannula piston pushes the flowable, therapeutic composition toward the distal end of the cannula for expressing the flowable, therapeutic composition.

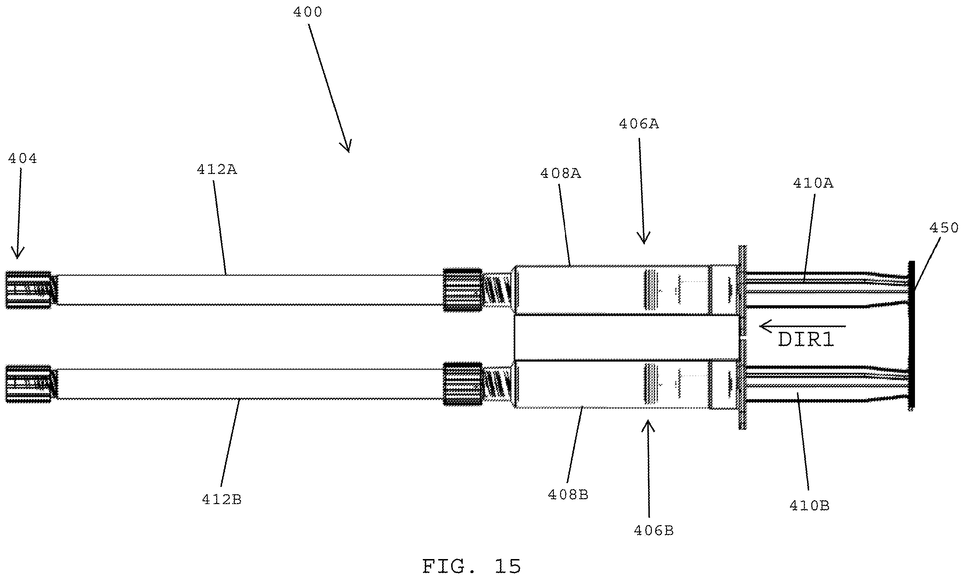

Referring to FIG. 15, in one embodiment, a system for delivering a therapeutic composition preferably includes a delivery device 400 having one or more of the features shown and described above in FIGS. 1A-14B. For example, although not shown, a first cannula piston is disposed within the first cannula 412A and a second cannula piston is disposed within the second cannula 412B. The cannula pistons may be similar to the cannula pistons shown and described in FIGS. 8A-14B of the present patent application.

In one embodiment, the delivery device 400 preferably has a two cannula configuration for dispensing two components that are mixed together to form the flowable, therapeutic composition (e.g., a flowable hemostat). The delivery device 400 preferably includes a first pump assembly 406A that is coupled with a first cannula 412A that contains a first component. The first pump assembly 406A preferably includes a first syringe barrel 408A and a first syringe plunger 410A that is coupled with the first syringe barrel 408A. The first syringe barrel 408A preferably includes a pumping fluid such as saline, water, or air. A first part of a two-part composition is disposed within the elongated lumen of the first cannula 412A.

The delivery device 400 preferably includes a second pump assembly 406B including a second syringe barrel 408B and a second syringe plunger 410B. The second syringe barrel 408B preferably includes a pumping fluid such as saline, water, or air. The delivery device 400 preferably includes a second cannula 412B that contains a second component that is mixed with the first component contained within the first cannula 412A. The two components that are disposed within the respective first and second cannulas 412A, 412B are preferably maintained apart from one another until they are dispensed simultaneously from the distal end 404 of the delivery device 400, whereupon they are mixed together to form the flowable, therapeutic composition.

In one embodiment, a thumb tab 450 preferably links the proximal ends of the first and second syringe plungers 410A, 410B, which ensures that the plungers are depressed simultaneously and/or together. The thumb tab 450 may be depressed in a distal direction DIR1 for forcing the pump fluid (e.g., saline) that is disposed within the respective first and second syringe barrels 408A, 408B into the proximal ends of the respective cannulas 4112A, 412B for dispensing the two components that fill the respective cannulas from the distal end 404 of the delivery device 400.

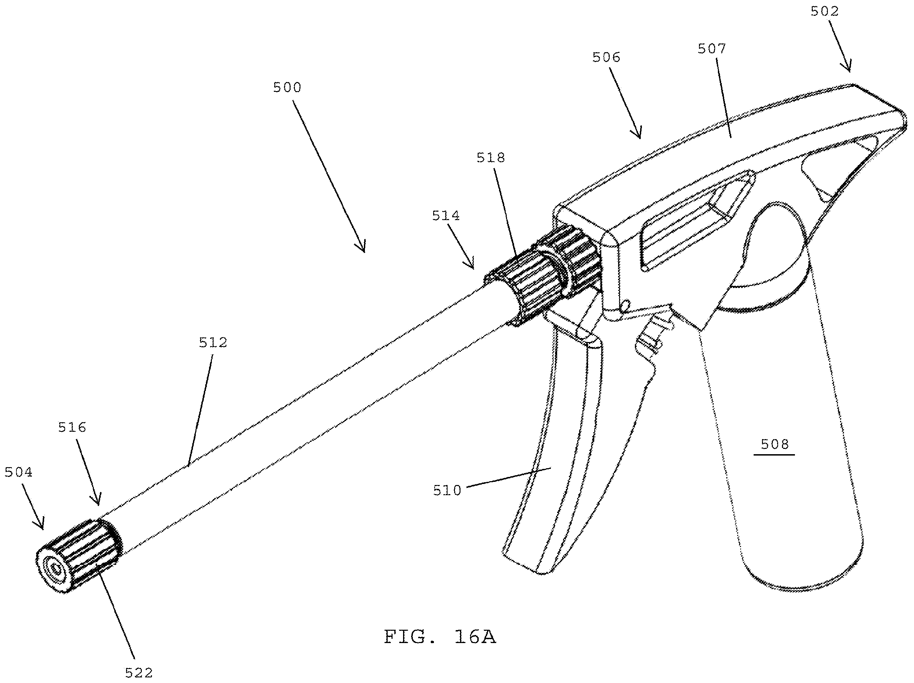

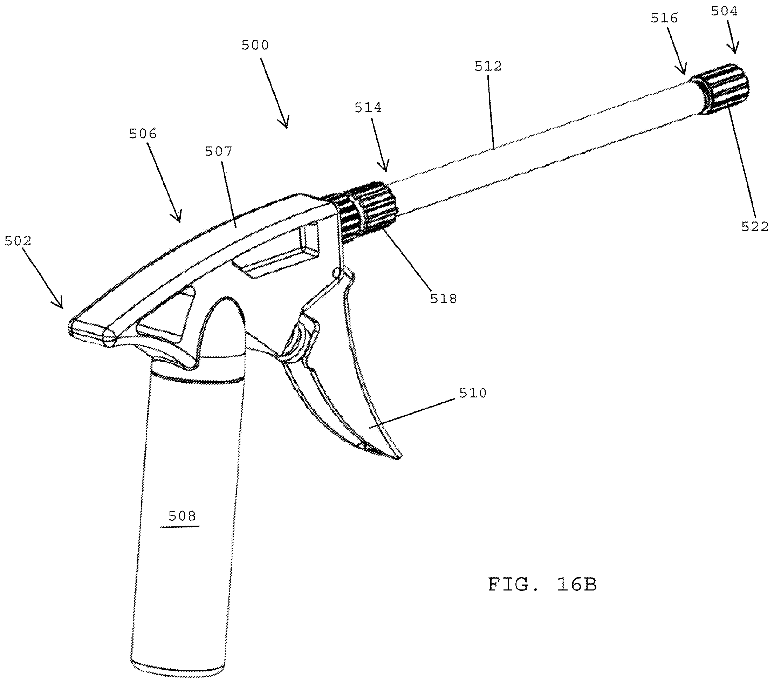

Referring to FIGS. 16A and 16B, in one embodiment, a system for dispensing a flowable, therapeutic composition (e.g., a flowable hemostat) preferably includes a delivery device 500 having one or more of the features shown and described above in FIGS. 1A-14B.

In one embodiment, the delivery device 500 preferably has a proximal end 502 and a distal end 504. The delivery device 500 preferably includes a pump assembly 506 including a pump assembly housing 507, a fluid reservoir 508 for containing a pumping fluid (e.g., saline; air), and a trigger 510 that may be squeezed for forcing the fluid contained within the fluid reservoir 508 to flow into a cannula 512 that contains a flowable, therapeutic composition. In one embodiment, the cannula 512 preferably includes a proximal end 514 that is coupled with a distal end of the housing 507 via a connector 518. A cannula piston (not shown) is preferably disposed within the elongated lumen of the cannula and preferably isolates the flowable, therapeutic composition that fills the cannula from the pumping fluid (e.g., saline) contained within the fluid reservoir 508 and the pump assembly 506.

According to the invention, the cannula 512 includes a distal end 516 that is configured for releasably securing an end cap 522 to the distal end of the cannula 512. The cannula 512 includes an elongated lumen that contains a flowable, therapeutic composition. According to the invention, end cap 522 may be removed from the distal end of the cannula for filling the elongated lumen of the cannula with the therapeutic composition. After the cannula has been filled with the therapeutic composition, the end cap may be re-secured to the distal end of the cannula for storage and/or shipment of the delivery device 500. In one embodiment not forming part of the claimed invention, during a surgical procedure, the end cap 522 is configured to be preferably removed from the distal end of the cannula prior to dispensing and/or expressing the therapeutic composition.

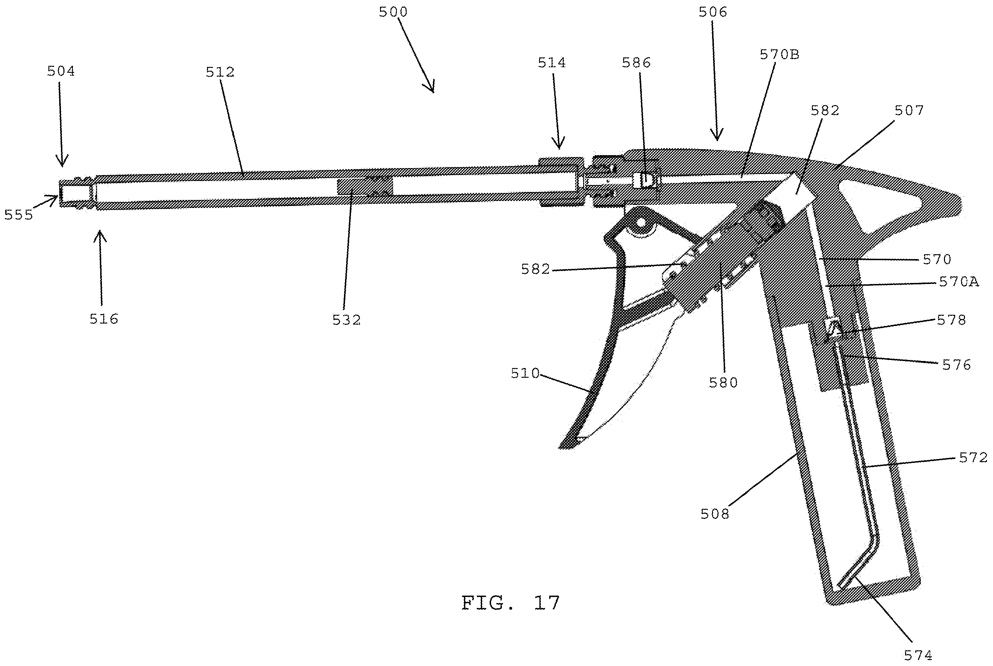

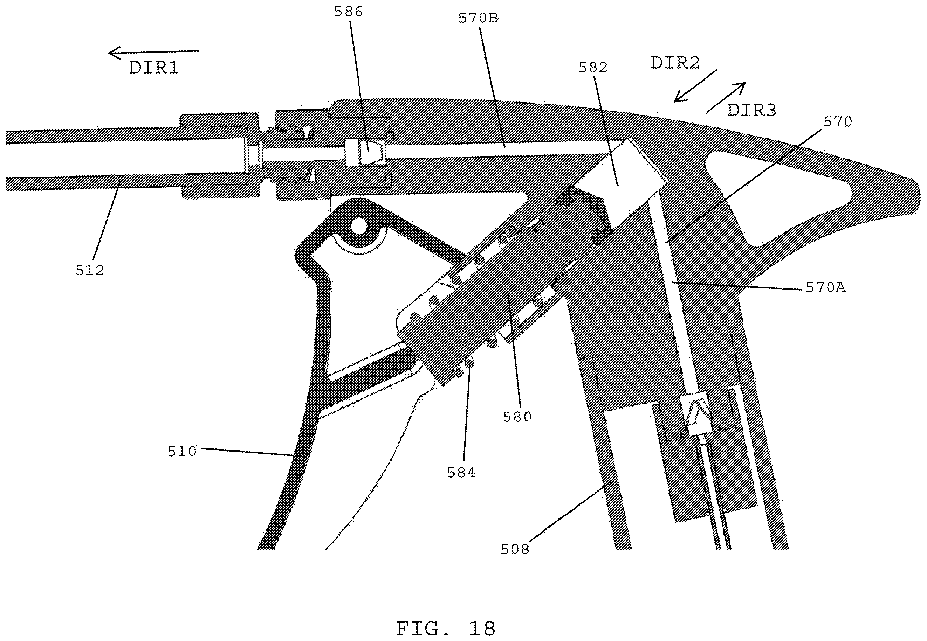

Referring to FIGS. 17 and 18, in one embodiment, the pump assembly 506 of the delivery device 500 preferably includes the pump assembly housing 507, the fluid reservoir 508 and the trigger 510 that may be squeezed for activating the pump assembly 506 to force the pumping fluid into the proximal end of the canula 512. In one embodiment, the trigger 510 preferably has an upper end that is pivotally connected with the pump assembly housing 507.

The pump assembly 506 preferably includes a fluid conduit 570 that extends between the fluid reservoir 508 and the proximal end 514 of the cannula 512. The pump assembly 506 desirably includes a suction tube 572 having a lower end 574 that is disposed within the fluid reservoir 508 and an upper end 576 that is in communication with the fluid conduit 570.

In one embodiment, the pump assembly 506 preferably includes a first one-way valve 578 that enables fluid within the fluid reservoir 508 to be drawn into the proximal end of the fluid conduit 570, however, the one-way valve 578 prevents the fluid from reversing direction and flowing from the fluid conduit 570 back into the fluid reservoir 508.

The pump assembly 506 preferably includes a piston 580 that is disposed within a pump chamber 582. A proximal end of the piston is coupled with the proximal side of the trigger 510. The pump assembly 506 preferably includes a spring 584 that is compressed when the trigger 510 is squeezed and that returns the trigger to an extended position when the trigger is released.

In one embodiment, the pump assembly 506 preferably includes a second one-way valve 586 that is located within a distal leg 570B of the fluid conduit 570, and which is located downstream from the pump chamber 582.

Referring to FIG. 17, in one embodiment, the delivery device 500 preferably includes a cannula 512 having a proximal end 514 and a distal end 516. The cannula preferably includes an elongated lumen 548 that extends between the proximal and distal ends thereof. A cannula piston 532 is preferably disposed within the elongated lumen 548 of the cannula 512. The cannula piston 532 preferably forms a seal with an inner surface of the cannula 512 and isolates a pumping fluid (e.g., saline) contained within the pump assembly 506 from a flowable, therapeutic composition contained within the elongated lumen 548 and located downstream of the cannula piston 532 (i.e., between the cannula piston 532 and the distal end 516 of the cannula 512).

The delivery device 500 preferably includes an end cap 522 that is releasably secured to the distal end 504 of the delivery device 500. In one embodiment, the end cap 522 preferably includes internal threads that mesh with external threads provided at the distal end 516 of the cannula 512 for releasably securing the end cap 522 to the distal end 516 of the cannula 512. The end cap 522 may be removed from the distal end of the cannular to fill the elongated lumen of the cannula with the flowable, therapeutic composition.

Referring to FIGS. 17 and 18, in one embodiment, as the spring 584 moves into an extended position, a lower end of the trigger 510 is pivoted away from the fluid reservoir 508 which retracts the piston 580 in the direction DIR2. As the piston 580 is retracted, a vacuum is created within the pump chamber 582 for drawing the fluid from the fluid reservoir 508, through an upstream leg 570A of the fluid conduit 570, and into the pump chamber 582. After fluid has filled the pump chamber 582, the trigger 510 may be squeezed for compressing the spring 584 and forcing the piston 580 in the direction DIR3, which, in turn, forces the fluid in the pump chamber 582 to flow into the downstream leg 570B of the fluid conduit 570.

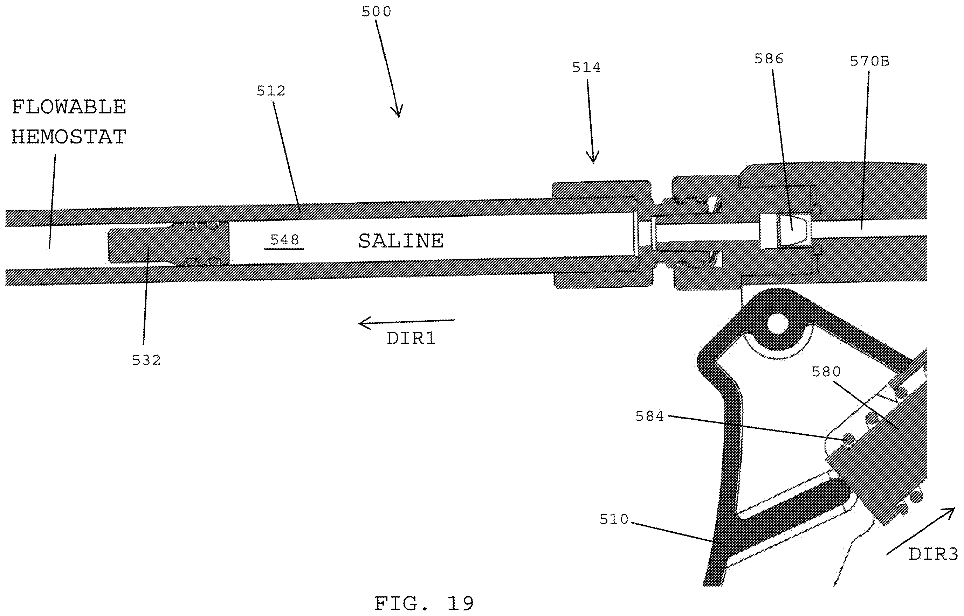

Referring to FIGS. 18 and 19, in one embodiment, when the trigger 510 is squeezed toward the fluid reservoir 508, the spring 584 is compressed as the piston 580 moves in the direction designated DIR3. The piston 580 forces the fluid in the pump chamber 582 to flow into the downstream leg 570B of the fluid conduit 570 and through the one-way valve 586 for being directed into the proximal end of the elongated lumen 548 of the cannula 512. The fluid that is directed into the proximal end of the elongated conduit of the cannula forces the cannula piston 532 to move in the distal direction DIR1 (i.e., toward the distal end of the cannula). The cannula piston 532 preferably isolates the saline fluid located on an upstream side of the cannula piston 532 from the flowable, therapeutic composition located on the downstream side of the cannula piston 532. As the saline fluid forces the cannula piston 532 to move in a distal direction DIR1, the piston pushes the flowable, therapeutic composition that fills the cannula toward the distal end of the delivery device 500 for dispensing the flowable, therapeutic composition from a dispensing opening 555 (FIG. 17) located at the distal end 504 of the delivery device 500.

While the foregoing is directed to embodiments of the present invention, other and further embodiments of the invention may be devised without departing from the basic scope thereof, which is only limited by the scope of the claims that follow. For example, the present invention contemplates that any of the features shown in any of the embodiments described herein be incorporated with any of the features shown in any of the other embodiments described herein, and still fall within the scope of the present invention, as long as the result falls within the scope of the appended claims.

## Claims

1. A system for delivering a flowable, therapeutic composition comprising:
a cannula (112; 212; 312; 512) having a proximal end (114; 514) with a proximal opening, a distal end (116; 216; 516) with a distal opening, and an elongated lumen (148; 248; 548) extending from the proximal end to the distal end;
a flowable, therapeutic composition filling the elongated lumen of said cannula;
a piston (232) disposed within the elongated lumen for sealing the proximal end of said cannula; wherein the piston includes a proximal section (134) and a distal section (136) having a distally extending nose (138; 238);
an end cap (122, 222) releasably secured to the distal end of said cannula; wherein the end cap functions as an applicator tip (222) for dispensing said therapeutic composition from the distal end of the cannula and has a constriction for preventing the piston from exiting the distal end of the cannula; wherein the end cap functioning as the applicator tip includes a dispensing channel (225) that is adapted to receive the distal nose of the piston;
a pump assembly (106; 506) including a fluid, said pump assembly being coupled with the proximal end of said cannula, wherein said pump assembly is operable to direct the fluid to flow through the proximal opening of said cannula to force said piston to slide toward the distal end of said cannula to express said flowable, therapeutic composition from the distal end of said cannula.

2. The system as claimed in claim 1, wherein said cannula comprises a flexible section (312B) that enables the distal end of said cannula to be flexed into a curved configuration.

3. The system as claimed in claim 1, wherein said cannula comprises a rigid section (312A) and a flexible section (312B).

4. The system as claimed in claim 1, wherein said flowable, therapeutic composition comprises a viscous fluid, and wherein the fluid of said pump assembly comprises saline or air having a lower viscosity than said viscous fluid.

5. The system as claimed in claim 1, wherein said flowable, therapeutic composition is a composition selected from the group consisting of flowable hemostats, biologics, and medicine paste.

6. The system as claimed in claim 1, wherein said piston has an outer perimeter that forms a seal with an inner surface (152) of said cannula.

7. The system as claimed in claim 6, wherein said piston comprises a cylindrical body and at least one O-ring (140A; 140B) coupled with said cylindrical body, said at least one O-ring having an outer perimeter that is configured to slide over the inner surface of said cannula for forming the seal with the inner surface of said cannula.

8. The system as claimed in claim 6, wherein said piston is selected from the group consisting of a cylindrical shaped body, a flexible body, a body made of rubber, and one or more balls.

9. The system as claimed in claim 1, wherein said pump assembly comprises:
a syringe including a syringe barrel (108) and a syringe plunger (110) that is disposed inside said syringe barrel;
a syringe connector (118; 518) having a proximal end coupled with a distal end (120; 507) of said syringe barrel and a distal end coupled with the proximal end of said cannula, wherein said syringe connector has a fluid conduit (570) that extends from the proximal end to the distal end of said syringe connector to define a fluid flow path between said syringe barrel and the proximal end of said cannula.

10. The system as claimed in claim 9, wherein depressing said syringe plunger toward the distal end of said syringe barrel expels said fluid from said syringe barrel and forces said fluid through said fluid flow path of said syringe connector and into the elongated lumen at the proximal end of said cannula.

11. The system as claimed in claim 1, wherein said pump assembly comprises:
a fluid reservoir (508) for storing the fluid of said pump assembly;
a pump chamber (582) that is in fluid communication with said fluid reservoir;
a plunger disposed within said pump chamber, wherein said plunger is moveable in a first direction for generating a vacuum within said pump chamber for drawing the fluid into said pump chamber and is moveable in a second direction for forcing the fluid from said pump chamber;
a squeezable trigger (510) coupled with said plunger for moving said plunger within said pump chamber.

## Patentansprüche

1. System zum Abgeben einer fließfähigen, therapeutischen Zusammensetzung, umfassend:
eine Kanüle (112; 212; 312; 512), die ein proximales Ende (114; 514) mit einer proximalen Öffnung, ein distales Ende (116; 216; 516) mit einer distalen Öffnung und ein längliches Lumen (148; 248; 548) aufweist, das sich von dem proximalen Ende zu dem distalen Ende erstreckt;
eine fließfähige, therapeutische Zusammensetzung, die das längliche Lumen der Kanüle füllt;
einen Kolben (232), der in dem länglichen Lumen angeordnet ist, zum Abdichten des proximalen Endes der Kanüle; wobei der Kolben einen proximalen Abschnitt (134) und einen distalen Abschnitt (136) mit einer sich distal erstreckenden Nase (138; 238) einschließt;
eine Endkappe (122, 222), die lösbar an dem distalen Ende der Kanüle befestigt ist; wobei die Endkappe als Applikatorspitze (222) zum Ausgeben der therapeutischen Zusammensetzung aus dem distalen Ende der Kanüle fungiert und eine Verengung aufweist, um ein Austreten des Kolbens aus dem distalen Ende der Kanüle zu verhindern; wobei die als Applikatorspitze fungierende Endkappe einen Ausgabekanal (225) einschließt, der angepasst ist, um die distale Nase des Kolbens aufzunehmen;
eine Pumpenbaugruppe (106; 506), die ein Fluid einschließt, wobei die Pumpenbaugruppe mit dem proximalen Ende der Kanüle gekoppelt ist, wobei die Pumpenbaugruppe betriebsfähig ist, um das Fluid zu leiten, um durch die proximale Öffnung der Kanüle zu fließen, um den Kolben zu zwingen, in Richtung des distalen Endes der Kanüle zu gleiten, um die fließfähige, therapeutische Zusammensetzung aus dem distalen Ende der Kanüle auszudrücken.

2. System nach Anspruch 1, wobei die Kanüle einen flexiblen Abschnitt (312B) umfasst, der es ermöglicht, dass das distale Ende der Kanüle in eine gekrümmte Konfiguration gebogen wird.

3. System nach Anspruch 1, wobei die Kanüle einen starren Abschnitt (312A) und einen flexiblen Abschnitt (312B) umfasst.

4. System nach Anspruch 1, wobei die fließfähige, therapeutische Zusammensetzung ein viskoses Fluid umfasst, und wobei das Fluid der Pumpenbaugruppe Kochsalzlösung oder Luft umfasst, die eine geringere Viskosität als das viskose Fluid aufweist.

5. System nach Anspruch 1, wobei die fließfähige, therapeutische Zusammensetzung eine Zusammensetzung ist, ausgewählt aus der Gruppe, bestehend aus fließfähigen Hämostatika, Biologika und Medizinpaste.

6. System nach Anspruch 1, wobei der Kolben einen Außenumfang aufweist, der eine Abdichtung mit einer inneren Oberfläche (152) der Kanüle ausbildet.

7. System nach Anspruch 6, wobei der Kolben einen zylindrischen Körper und mindestens einen mit dem zylindrischen Körper gekoppelten O-Ring (140A; 140B) umfasst, wobei der mindestens eine O-Ring einen Außenumfang aufweist, der konfiguriert ist, um über die innere Oberfläche der Kanüle zu gleiten, um die Abdichtung mit der inneren Oberfläche der Kanüle auszubilden.

8. System nach Anspruch 6, wobei der Kolben aus der Gruppe ausgewählt ist, bestehend aus einem zylinderförmigen Körper, einem flexiblen Körper, einem Körper hergestellt aus Gummi und einer oder mehreren Kugeln.

9. System nach Anspruch 1, wobei die Pumpenbaugruppe umfasst:
eine Spritze, einschließlich eines Spritzenzylinders (108) und eines Spritzenkolbens (110), der in dem Spritzenzylinder angeordnet ist;
einen Spritzenverbinder (118; 518), der ein proximales Ende, das mit einem distalen Ende (120; 507) des Spritzenzylinders gekoppelt ist, und ein distales Ende, das mit dem proximalen Ende der Kanüle gekoppelt ist, aufweist, wobei der Spritzenverbinder eine Fluidleitung (570), die sich von dem proximalen Ende zu dem distalen Ende des Spritzenverbinders erstreckt, um einen Fluidströmungsweg zwischen dem Spritzenzylinder und dem Hohlrohr zu definieren, aufweist.

10. System nach Anspruch 9, wobei das Herunterdrücken des Spritzenkolbens in Richtung des distalen Endes des Spritzenzylinders das Fluid aus dem Spritzenzylinder ausstößt und das Fluid durch den Fluidströmungsweg des Spritzenverbinders und in das längliche Lumen an dem proximalen Ende der Kanüle drückt.

11. System nach Anspruch 1, wobei die Pumpenbaugruppe umfasst:
ein Fluidreservoir (508) zum Lagern des Fluids der Pumpenbaugruppe;
eine Pumpenkammer (582), die in Fluidverbindung mit dem Fluidreservoir steht;
einen Kolben, der in der Pumpenkammer angeordnet ist, wobei der Kolben in eine erste Richtung zum Erzeugen eines Vakuums in der Pumpenkammer zum Ansaugen des Fluids in die Pumpenkammer beweglich ist und in eine zweite Richtung zum Herausdrücken des Fluids aus der Pumpenkammer beweglich ist;
einen zusammendrückbaren Auslöser (510), der mit dem Kolben gekoppelt ist, zum Bewegen des Kolbens innerhalb der Pumpenkammer.

## Revendications

1. Système permettant de délivrer une composition thérapeutique apte à l'écoulement comprenant :
une canule (112 ; 212 ; 312 ; 512) ayant une extrémité proximale (114 ; 514) avec une ouverture proximale, une extrémité distale (116 ; 216 ; 516) avec une ouverture distale, et une lumière allongée (148 ; 248 ; 548) s'étendant de l'extrémité proximale à l'extrémité distale ;
une composition thérapeutique apte à l'écoulement remplissant la lumière allongée de ladite canule ;
un piston (232) disposé au sein de la lumière allongée permettant d'assurer l'étanchéité de l'extrémité proximale de ladite canule ; dans lequel le piston comporte une section proximale (134) et une section distale (136) ayant un nez (138 ; 238) s'étendant distalement ;
une coiffe d'extrémité (122, 222) fixée de manière libérable à l'extrémité distale de ladite canule ; dans lequel la coiffe d'extrémité fonctionne en tant qu'embout applicateur (222) permettant de distribuer ladite composition thérapeutique à partir de l'extrémité distale de la canule et a un étranglement destiné à empêcher le piston de sortir de l'extrémité distale de la canule ; dans lequel la coiffe d'extrémité fonctionnant en tant qu'embout applicateur comporte un canal de distribution (225) qui est conçu pour recevoir le nez distal du piston ;
un ensemble pompe (106 ; 506) comportant un fluide, ledit ensemble pompe étant accouplé à l'extrémité proximale de ladite canule, dans lequel ledit ensemble pompe est fonctionnel pour diriger le fluide pour qu'il s'écoule à travers l'ouverture proximale de ladite canule pour forcer ledit piston à coulisser en direction de l'extrémité distale de ladite canule pour expulser ladite composition thérapeutique apte à l'écoulement à partir de l'extrémité distale de ladite canule.

2. Système selon la revendication 1, dans lequel ladite canule comprend une section flexible (312B) qui permet à l'extrémité distale de ladite canule d'être fléchie en une configuration courbée.

3. Système selon la revendication 1, dans lequel ladite canule comprend une section rigide (312A) et une section flexible (312B).

4. Système selon la revendication 1, dans lequel ladite composition thérapeutique apte à l'écoulement comprend un fluide visqueux, et dans lequel le fluide dudit ensemble pompe comprend une solution saline ou de l'air ayant une viscosité plus basse que ledit fluide visqueux.

5. Système selon la revendication 1, dans lequel ladite composition thérapeutique apte à l'écoulement est une composition choisie dans le groupe constitué de compositions hémostatiques, produits biologiques et pâte médicinale, aptes à l'écoulement.

6. Système selon la revendication 1, dans lequel ledit piston a un périmètre externe qui forme un joint d'étanchéité avec une surface interne (152) de ladite canule.

7. Système selon la revendication 6, dans lequel ledit piston comprend un corps cylindrique et au moins un joint torique (140A ; 140B) accouplé audit corps cylindrique, ledit au moins un joint torique ayant un périmètre externe qui est conçu pour coulisser par-dessus la surface interne de ladite canule pour la formation du joint d'étanchéité avec la surface interne de ladite canule.

8. Système selon la revendication 6, dans lequel ledit piston est choisi dans le groupe constitué d'un corps de forme cylindrique, d'un corps flexible, d'un corps fabriqué en caoutchouc, et d'une ou plusieurs billes.

9. Système selon la revendication 1, dans lequel ledit ensemble pompe comprend :
une seringue comportant un cylindre de seringue (108) et un mécanisme coulissant de seringue (110) qui est disposé à l'intérieur dudit cylindre de seringue ;
un raccord de seringue (118 ; 518) ayant une extrémité proximale accouplée à une extrémité distale (120 ; 507) dudit cylindre de seringue et une extrémité distale accouplée à l'extrémité proximale de ladite canule, dans lequel ledit raccord de seringue a un conduit de fluide (570) qui s'étend de l'extrémité proximale à l'extrémité distale dudit raccord de seringue pour définir un chemin d'écoulement de fluide entre ledit cylindre de seringue et l'extrémité proximale de ladite canule.

10. Système selon la revendication 9, dans lequel une pression sur ledit mécanisme coulissant de seringue en direction de l'extrémité distale dudit cylindre de seringue expulse ledit fluide dudit cylindre de seringue et force ledit fluide à travers ledit chemin d'écoulement de fluide dudit raccord de seringue et dans la lumière allongée au niveau de l'extrémité proximale de ladite canule.

11. Système selon la revendication 1, dans lequel ledit ensemble pompe comprend :
un réservoir de fluide (508) permettant de stocker le fluide dudit ensemble pompe ;
une chambre de pompe (582) qui est en communication fluidique avec ledit réservoir de fluide ;
un mécanisme coulissant disposé au sein de ladite chambre de pompe, dans lequel ledit mécanisme coulissant peut être déplacé dans une première direction pour la génération d'un vide au sein de ladite chambre de pompe permettant d'aspirer le fluide dans ladite chambre de pompe et peut être déplacé dans une seconde direction permettant de forcer le fluide à partir de ladite chambre de pompe ;
un déclencheur compressible (510) accouplé audit mécanisme coulissant permettant de déplacer ledit mécanisme coulissant au sein de ladite chambre de pompe.
